# EUROPEAN PATENT APPLICATION

(11) **EP 1 847 521 A1**
(43) Date of publication of application: **24.10.2007**
(21) Application number: 06711911.5
(22) Date of filing: 18.01.2006
(51) Int. Cl.: C07C 15/28, C09K 11/06, H01L 27/32

(54) **BISANTHRACENE DERIVATIVE AND ORGANIC ELECTROLUMINESCENT DEVICE USING SAME**

(30) Priority: 10.02.2005 JP 2005033767
(71) Applicant: IDEMITSU KOSAN CO., LTD., Tokyo 100-8321 (JP)
(72) Inventor: KUBOTA, Mineyuki, Chiba, 2990293 (JP); FUNAHASHI, Masakazu, Chiba, 2990293 (JP)
(74) Representative: Gille Hrabal Struck Neidlein Prop Roos
(86) International application number: PCT/JP2006/300642
(87) International publication number: WO 2006/085434

(57) **Abstract**

A bisanthracene derivative having a specific structure and an organic electroluminescence device having an organic thin film layer which has one layer or a plurality of layers including at least a light emitting layer, and is disposed between a cathode and an anode and contains the bisanthracene derivatives singly or as a component of a mixture. The organic electroluminescence device exhibits a great efficiency of light emission in a region including a high luminance region and has a long life, and the bisanthracene derivative realizes the device.

## Description

### TECHNICAL FIELD

The present invention relates to a bisanthracene derivative and an organic electroluminescence device using the derivative. More particularly, the present invention relates to an organic electroluminescence device exhibiting a great efficiency of light emission in a region including a high luminance region and having a long life and a bisanthracene derivative for realizing the device.

### BACKGROUND ART

An organic electroluminescence ("electroluminescence" will be referred to as "EL", hereinafter) device is a spontaneous light emitting device which utilizes the principle that a fluorescent substance emits light by energy of recombination of holes injected from an anode and electrons injected from a cathode when an electric field is applied. Since an organic EL device of the laminate type driven under a low electric voltage was reported by C. W. Tang of Eastman Kodak Company (C. W. Tang and S. A. Vanslyke, Applied Physics Letters, Volume 51, Pages 913, 1987), many studies have been conducted on organic EL devices using organic materials as the constituting materials. Tang et al. used a laminate structure using tris(8-hydroxyquinolinolato)aluminum for the light emitting layer and a triphenyldiamine derivative for the hole transporting layer. Advantages of the laminate structure are that the efficiency of hole injection into the light emitting layer can be increased, that the efficiency of forming excitons which are formed by blocking electrons injected from the cathode and recombining can be increased, and that excited particles formed within the light emitting layer can be enclosed. As the structure of the organic EL device, a two-layered structure having a hole transporting (injecting) layer and an electron transporting and light emitting layer and a three-layered structure having a hole transporting (injecting) layer, a light emitting layer and an electron transporting (injecting) layer are well known. To increase the efficiency of recombination of injected holes and electrons in the devices of the laminate type, the structure of the device and the process for forming the device have been studied.

As the light emitting material of the organic EL device, chelate complexes such as tris(8-quinolinolato)aluminum, coumarine derivatives, tetraphenylbutadiene derivatives, bistyrylarylene derivatives and oxadiazole derivatives are known. It is reported that light in the visible region ranging from blue light to red light can be obtained by using these light emitting materials, and development of a device exhibiting color images is expected (For example, Patent Reference 1, Patent Reference 2 and Patent Reference 3).

Devices using a bisanthracene derivative as the light emitting material are disclosed in Patent References 4 to 7. The bisanthracene derivatives are used as the material emitting blue light, and has drawbacks in that the life of the device is not sufficient, and that the efficiency of light emission decreases in the high luminance region. In particular, the increase in the life and the increase in the efficiency in the high luminance region of passive matrix driving devices have been required since the passive matrix driving devices are driven in the high luminance region
[Patent Reference 1] Japanese Patent Application Laid-Open No. Heisei 8(1996)-239655
[Patent Reference 2] Japanese Patent Application Laid-Open No. Heisei 7(1995)-138561
[Patent Reference 3] Japanese Patent Application Laid-Open No. Heisei 3(1991)-200289
[Patent Reference 4] Japanese Patent Application Laid-Open No. Heisei 8(1996)-12600
[Patent Reference 5] Japanese Patent Application Laid-Open No. 2000-344691
[Patent Reference 6] Japanese Patent Application Laid-Open No. 2004-2351
[Patent Reference 7] Japanese Patent Application Laid-Open No. 2005-15420

### DISCLOSURE OF THE INVENTION

The present invention has been made to overcome the above problems and has an object of providing an organic EL device exhibiting a great efficiency of light emission in a region including a high luminance region and having a long life and a novel bisanthracene derivative for realizing the device.

As the result of intensive studies by the present inventors to achieve the above object, it was found that an organic EL device exhibiting a great efficiency of light emission in a region including a high luminance region and having a long life could be obtained when a bisanthracene derivative having a specific structure represented by general formula (1) shown below was used as a material of an organic thin film layer in an organic EL device. The present invention has been completed based on the knowledge.

The present invention provides a bisanthracene derivative represented by following general formula (1): 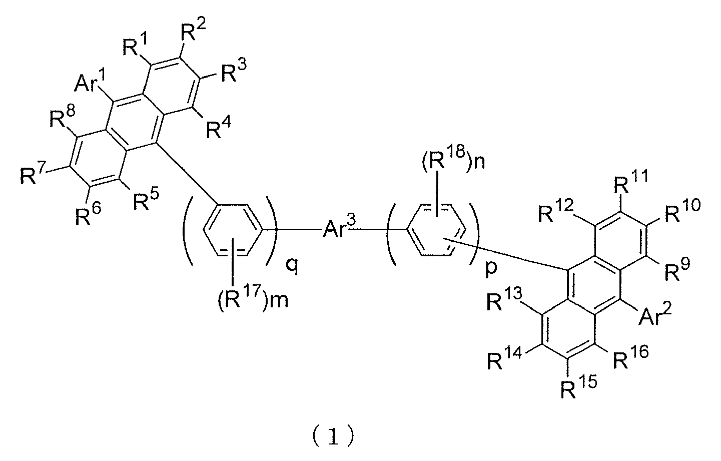 wherein Ar¹ and Ar² each independently represent a substituted or unsubstituted aromatic hydrocarbon group having 6 to 50 nuclear carbon atoms, and Ar³ represents a substituted or unsubstituted phenylene group, naphthylene group, chrysenylene group, biphenylene group or fluorenylene group ;
R¹ to R¹⁸ each independently represent hydrogen atom, a substituted or unsubstituted aromatic hydrocarbon group having 6 to 50 nuclear carbon atoms, a substituted or unsubstituted aromatic heterocyclic group having 5 to 50 nuclear atoms, a substituted or unsubstituted alkyl group having 1 to 50 carbon atoms, a substituted or unsubstituted cycloalkyl group having 3 to 50 nuclear carbon atoms, a substituted or unsubstituted alkoxyl group having 1 to 50 carbon atoms, a substituted or unsubstituted aralkyl group having 6 to 50 carbon atoms, a substituted or unsubstituted aryloxyl group having 5 to 50 nuclear carbon atoms, a substituted or unsubstituted arylthio group having 5 to 50 nuclear carbon atoms, a substituted or unsubstituted alkoxycarbonyl group having 1. to 50 carbon atoms, a substituted or unsubstituted silyl group, carboxyl group, a halogen atom, cyano group, nitro group or hydroxyl group;
m and n each represent an integer of 0 to 4 and, when m and n each represent an integer of 2 or greater, atoms and groups represented by R¹⁷ and R¹⁸, respectively, may be a same with or different from each other and may be bonded to each other to form a cyclic structure; and
q represents an integer of 1 to 3, and p represents an integer of 0 to 2.

The present invention also provides an organic electroluminescence device comprising a cathode, an anode and an organic thin film layer which comprises one layer or a plurality of layers comprising at least a light emitting layer and is disposed between the cathode and the anode, wherein the organic thin film layer comprises at least one compound selected from bisanthracene derivatives described above singly or as a component of a mixture.

### EFFECT OF THE INVENTION

The organic EL device comprising the bisanthracene derivative of the present invention exhibits a great efficiency of light emission in a region including a high luminance region and has a long life.

### THE MOST PREFERRED EMBODIMENT TO CARRY OUT THE INVENTION

The bisanthracene derivative of the present invention is a compound represented by the following general formula (1):

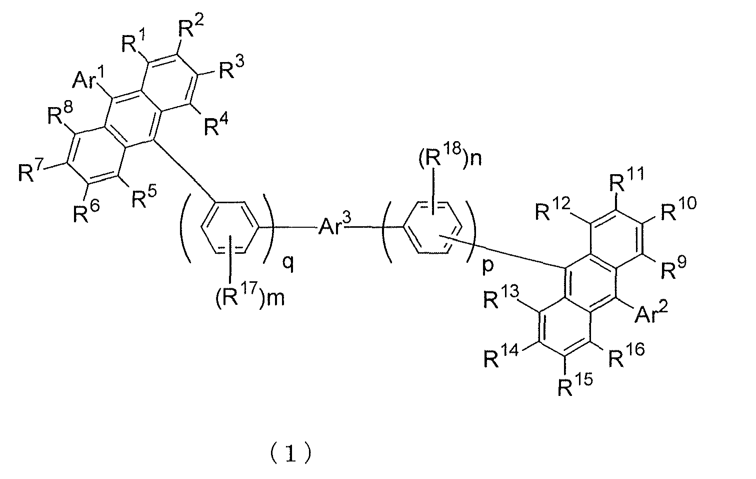

In general formula (1), Ar¹ and Ar² each independently represent a substituted or unsubstituted aromatic hydrocarbon group having 6 to 50 nuclear carbon atoms.

Examples of the aromatic hydrocarbon group represented by Ar¹ and Ar² include phenyl group, 1-naphthyl group, 2-naphthyl group, 1-anthryl group, 2-anthryl group, 9-anthryl group, 9-(10-phenyl)anthryl group, 9-(10-naphthyl-1-yl)anthryl group, 9-(10-naphthyl-2-yl)anthryl group, 1-phenanthryl group, 2-phenanthryl group, 3-phenanthryl group, 4-phenanthryl group, 9-phenanthryl group, 6-chrysenyl, group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, 3-methyl-2-naphthyl group, 4-methyl-1-naphthyl group and 4-methyl-1-anthryl group.

Among these groups, phenyl group, 1-naphthyl group, 2-naphthyl group, 9-(10-phenyl)anthryl group, 9-(10-naphthyl-1-yl)anthryl group, 9-(10-naphthyl-2-yl)anthryl group, 9-phenanthryl group, 1-pyrenyl group, 2-pyrenyl group, 4-pyrenyl group, 2-biphenylyl group, 3-biphenylyl group, 4-biphenylyl group, o-tolyl group, m-tolyl group, p-tolyl group and p-t-butylphenyl group are preferable.

In general formula (1), Ar³ represents a substituted or unsubstituted phenylene group, naphthylene group, chrysenylene group, biphenylene group or fluorenylene group. A substituted or unsubstituted m-phenylene group and a substituted or unsubstituted naphthylene group are preferable.

Examples of the substituent to the group represented by Ar¹ to Ar³ include alkyl groups (such as methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxy-isopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group, cyclopropyl group, cyclobutyl group, cyclopentyl group, cyclohexyl group, 4-methylcyclohexyl group, 1-adamantyl group, 2-adamantyl group, 1-norbornyl group and 2-norbornyl group), alkoxyl groups having 1 to 6 carbon atoms (such as ethoxyl group, methoxyl group, i-propoxyl group, n-propoxyl group, s-butoxyl group, t-butoxyl group, pentoxyl group, hexyloxyl group, cyclopentoxyl group and cyclohexyloxyl group), aryl groups having 5 to 40 nuclear atoms, amino groups substituted with aryl groups having 5 to 40 nuclear atoms, ester groups having aryl groups having 5 to 40 nuclear atoms, ester groups having alkyl groups having 1 to 6 carbon atoms, cyano group, nitro group and halogen atoms.

In general formula (1), R¹ to R¹⁸ each independently represent hydrogen atom, a substituted or unsubstituted aromatic hydrocarbon group having 6 to 50 nuclear carbon atoms, a substituted or unsubstituted aromatic heterocyclic group having 5 to 50 nuclear atoms, a substituted or unsubstituted alkyl group having 1 to 50 carbon atoms, a substituted or unsubstituted cycloalkyl group having 3 to 50 nuclear carbon atoms, a substituted or unsubstituted alkoxyl group having 1 to 50 carbon atoms, a substituted or unsubstituted aralkyl group having 6 to 50 carbon atoms, a substituted or unsubstituted aryloxyl group having 5 to 50 nuclear carbon atoms, a substituted or unsubstituted arylthio group having 5 to 50 nuclear carbon atoms, a substituted or unsubstituted alkoxycarbonyl group having 1 to 50 carbon atoms, a substituted or unsubstituted silyl group, carboxyl group, a halogen atom, cyano group, nitro group or hydroxyl group.

Examples of the substituted and unsubstituted aromatic hydrocarbon groups represented by R¹ to R¹⁸ include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group and 4"-t-butyl-p-terphenyl-4-yl group.

Examples of the substituted and unsubstituted aromatic heterocyclic groups represented by R¹ to R¹⁸ include 1-pyrrolyl group, 2-pyrrolyl group, 3-pyrrolyl group, pyradinyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxanyl group, 5-quinoxanyl group, 6-quinoxanyl group, 1-carbazolyl group, 2-carbazolyl group, 3-carbazolyl group, 4-carbazolyl group, 9-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 10-phenothiazinyl group, 1-phenoxazinyl group, 2-phenoxazinyl group, 3-phenoxazinyl group, 4-phenoxazinyl group, 10-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methyl-pyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group and 4-t-butyl-3-indolyl group.

Examples of the substituted and unsubstituted alkyl groups represented by R¹ to R¹⁸ include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxy-isopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodolsopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3- tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group.

Examples of the substituted and unsubstituted cycloalkyl groups represented by R¹ to R¹⁸ include cyclopropyl group, cyclobutyl group, cyclopentyl group, cyclohexyl group, 4-methylcyclohexyl group, 1-adamantyl group, 2-adamantyl group, 1-norbornyl group and 2-norbornyl group.

The substituted or unsubstituted alkoxyl group represented by R¹ to R¹⁸ is a group represented by -OY. Examples of the group represented by Y include the groups described as the examples of the alkyl group.

Examples of the substituted and unsubstituted aralkyl groups represented by R¹ to R¹⁸ include benzyl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthyl-isopropyl group, β-naphthylanethyl group, 1-β-naphthylethyl group, 2-β-naphthylethyl group, 1-β-naphthylisopropyl group, 2-β-naphthyl-isopropyl group, 1-pyrrolylmethyl group, 2-(1-pyrrolyl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group and 1-chloro-2-phenylisopropyl group.

The substituted or unsubstituted aryloxyl group represented by R¹ to R¹⁸ is a group represented by -OY'. Examples of the group represented by Y' include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4"-t-butyl-p-terphenyl-4-yl group, 2-pyrrolyl group, 3-pyrrolyl group, pyradinyl group, 2-pyridinyl group, 3-pyridinyl group, 4-pyridinyl group, 2-indolyl group, 3-indolyl group, 4-indolyl group, 5-indolyl group, 6-indolyl group, 7-indolyl group, 1-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxanyl group, 5-quinoxanyl group, 6-quinoxanyl group, 1-carbazolyl group, 2-carbazolyl group, 3-carbazolyl group, 4-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1, 10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8 phenanthrolin-4-yl group, 2,8 phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 1-phenoxazinyl group, 2-phenoxazinyl group, 3-phenoxazinyl group, 4-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrol-5-yl group, 3-methylpyrrol-1-yl group, 3-methyl-pyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl- 3-indolyl group and 4-t-butyl-3-indolyl group.

The substituted or unsubstituted arylthio group represented by R¹ to R¹⁸ is represented by -SY'. Examples of the group represented by Y' include the groups described above as the examples of the group represented by Y' in the aryloxyl group.

The substituted or unsubstituted alkoxycarbonyl group represented by R¹ to R¹⁸ is represented by -COOZ. Examples of the group represented by Z include the groups described above as the examples of the alkyl group.

Examples of the substituted or unsubstituted silyl group represented by R¹ to R¹⁸ include trimethylsilyl group, triethylsilyl group, t-butyldimethylsilyl group, vinyldimethylsilyl group and propyldimethylsilyl group.

Examples of the halogen atom represented by R¹ to R¹⁸ include fluorine atom, chlorine atom, bromine atom and iodine atom.

Examples of the substituent to the group represented by R¹ to R¹⁸ include the substituents described as the examples of the substituent to the group represented by Ar¹ to Ar³.

In general formula (1), m and n each represent an integer of 0 to 4 and preferably an integer of 0 to 2.

When m and n each represent an integer of 2 or greater, atoms and groups represented by R¹⁷ and R¹⁸, respectively, may be the same with or different from each other and may be bonded to each other to form a cyclic structure,

Examples of the above cyclic structure include structures derived from cycloalkanes having 4 to 12 carbon atoms such as cyclobutane, cyclopentane, cyclohexane, admantane and norbornane; cycloalkenes having 4 to 12 carbon atoms such as cyclobutene, cyclpentene, cyclohexene, cycloheptene and cyclooctene; cycloalkadienes having 6 to 12 carbon atoms such as cyclohexadiene, cycloheptadiene and cyclooctadiene; aromatic rings having 6 to 50 carbon atoms such as benzene, naphthalene, phenanthrene, anthracene, pyrene, chrysene and acenaphthylene; and hetero rings having 5 to 50 carbon atoms such as imidazole, pyrrol, furan, thiophene and pyridine.

In general formula (1), q represents an integer of 1 to 3 and preferably 1 or 2, and p represents an integer of 0 to 2 and preferably 0 or 1.

Bisanthracene derivatives represented by general formula (1) in which (i) q represents 1 and Ar³ represents phenylene group, (ii) q represents 1, p represents 0 or 1, and Ar³ represents phenylene group, or (iii) q represents 1, and Ar³ represents naphthylene group, are preferable. Among these bisanthracene derivatives, bisanthracene derivatives represented by general formula in which Ar³ represents m-phenylene group or naphthylene group are more preferable.

Examples of the bisanthracene derivatives represented by general formula (1) of the present invention are shown in the following. However, the bisanthracene derivative of the present invention is not limited to these compounds.

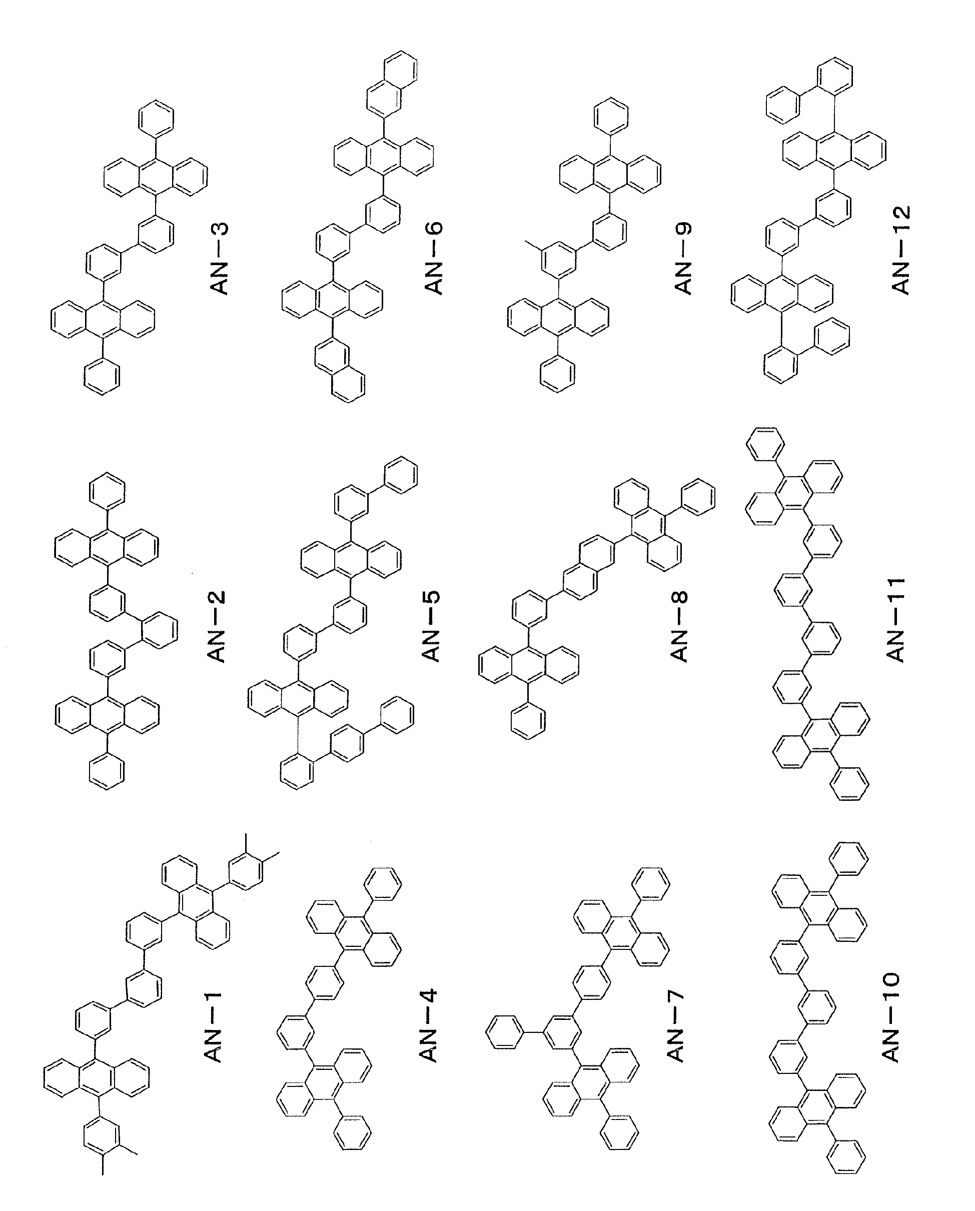

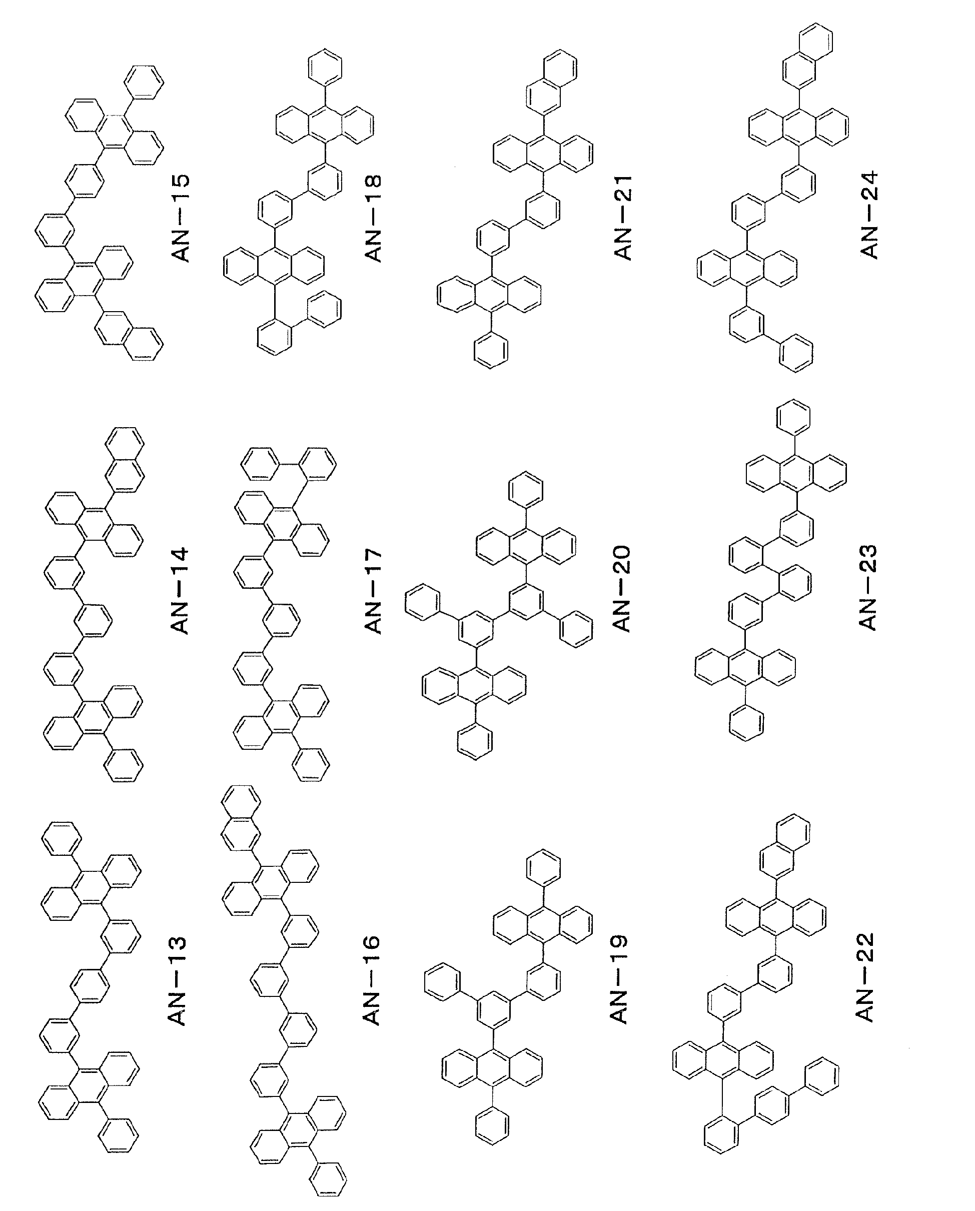

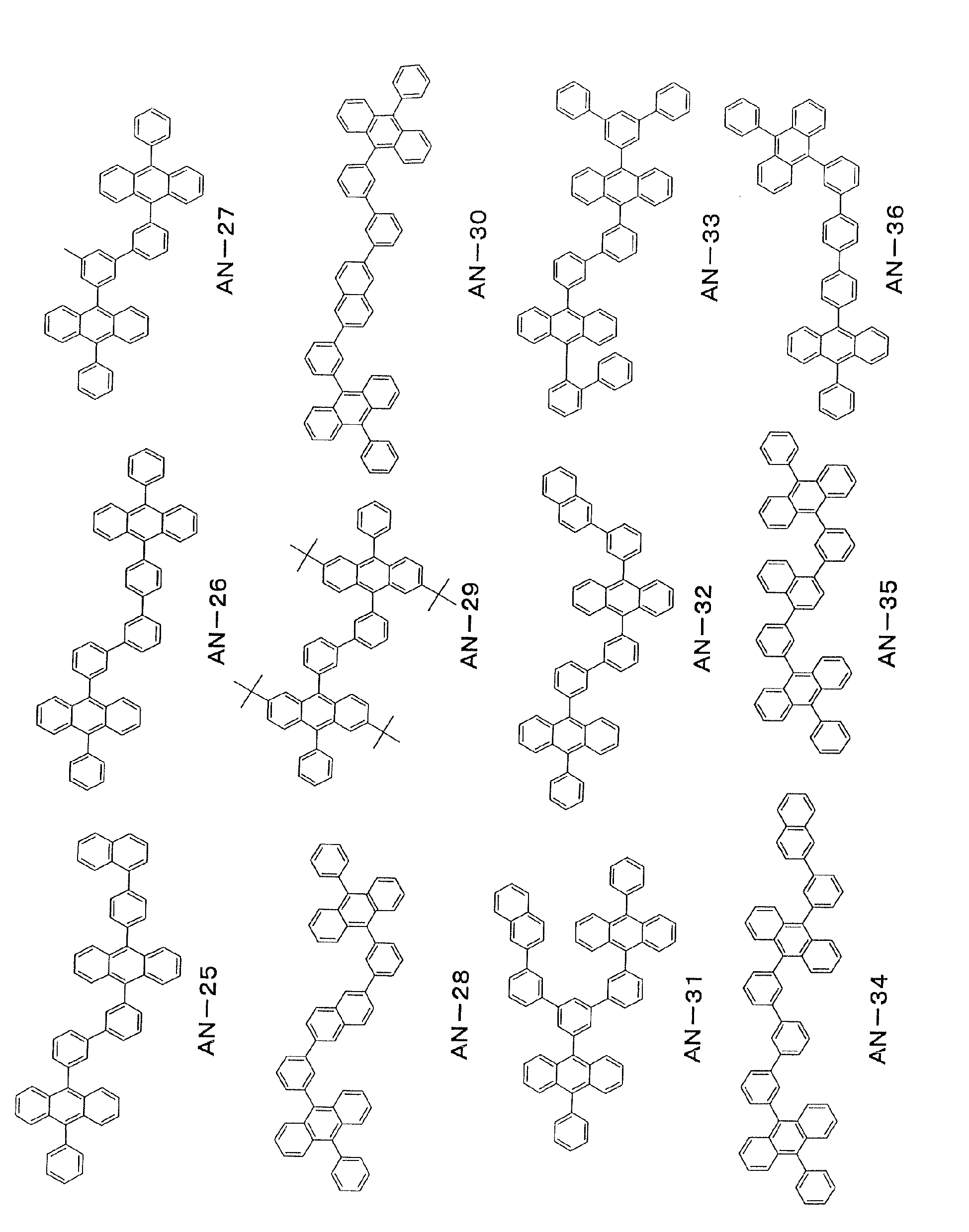

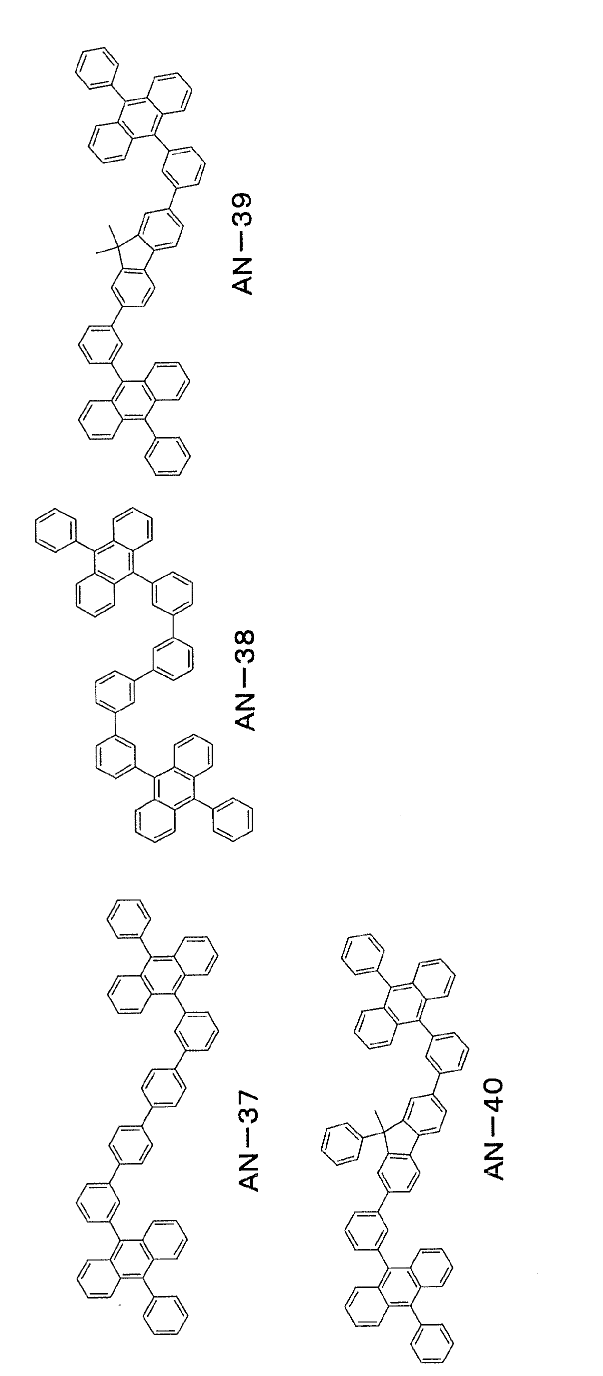

The process for producing the bisanthracene derivative of the present invention will be described in the following. The bisanthracene derivative of the present invention can be produced, for example, in accordance with the following synthetic scheme.

In the following scheme, Ar¹, Ar², Ar³, R¹ to R¹⁸, m, n, p and q are each as defined above. X¹ and X² each represent a halogen atom. R¹⁹ to R²⁴ each independently represent hydroxyl group or an alkoxyl group which may have substituents, and adjacent groups represented by R¹⁹ to R²⁴ may be bonded to each other to form a cyclic structure. Tf represents trofluoromethanesulfonyl group, and Ts represents p-toluenesulfonyl group.

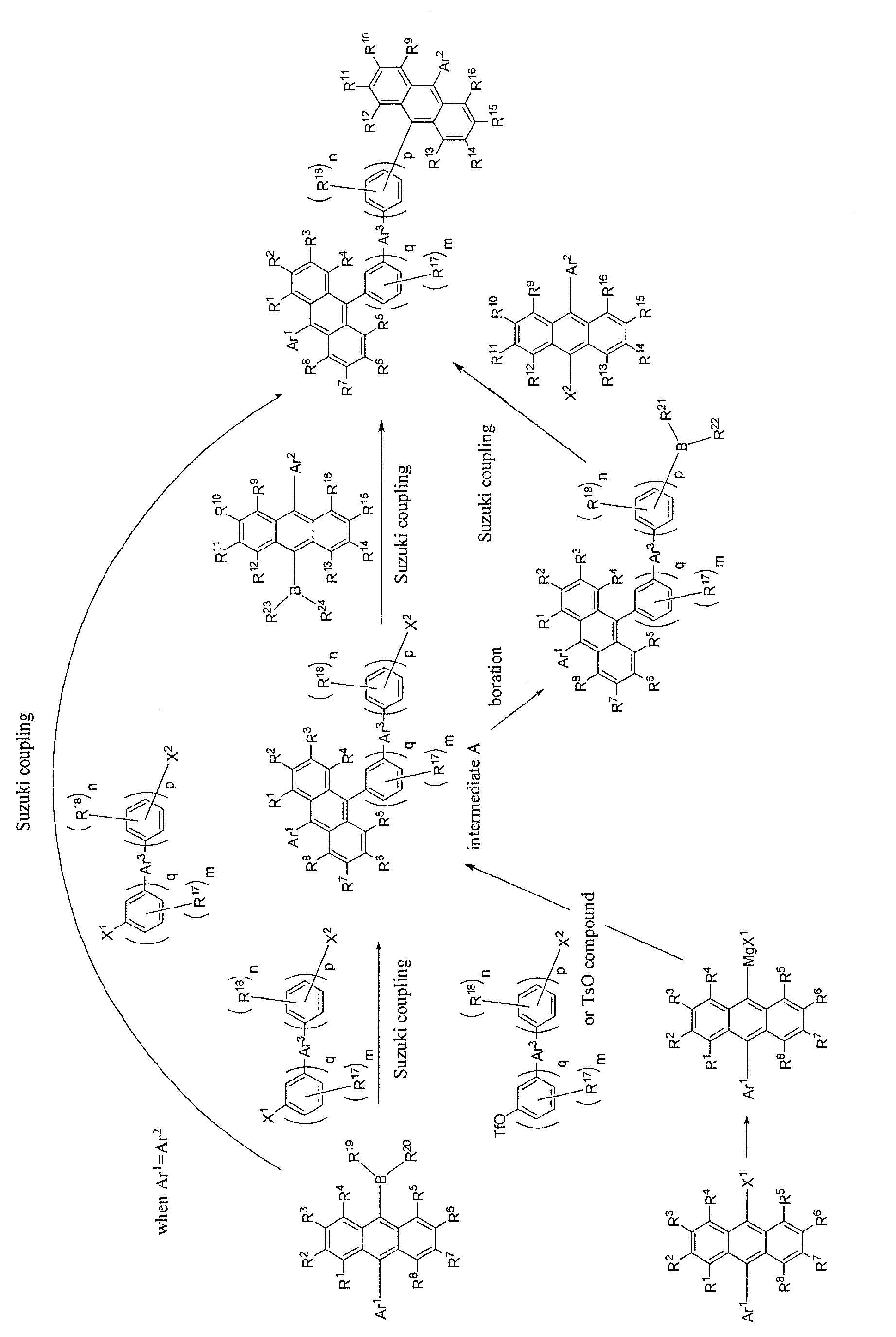

As shown in the above scheme, the above bisanthracene derivative can be obtained by a reaction such as the Suzuki coupling reaction using an anthraceneboronic acid derivative or a halogenated anthracene derivative synthesized in accordance with a conventional process as the starting material. In particular, a bisanthracene derivative represented by general formula (1) in which Ar¹≠ Ar² (Ar¹ and Ar² represent different groups) can be obtained easily with a high purity by a stepwise synthesis using a halogen compound having two or more reaction points of different reactivities. Removal of impurities can be easily achieved when the step of isolation and purification of intermediate A is inserted although the isolation of intermediate A is not always necessary. Intermediate A can be obtained also by reacting a Grignard compound derived from a halogenated anthracene derivative with a halogen compound having two or more reaction points of different reactivities such as a halogenated aryl triflate or a halogenated aryl tosylate. A bisanthracene derivative represented by general formula (1) in which Ar¹=Ar² (Ar¹ and Ar² represent the same group) can be obtained in less steps by bringing an anthraceneboronic acid derivative having an equivalence of two or greater into reaction with a dihalogen compound in "one-pot" although the compound can be obtained in accordance with the above stepwise synthesis.

Many reports are found on the Suzuki coupling reaction (Chem. Rev. Vol. 95, No. 7, 2457 (1995) and others). The reaction can be conducted under the conditions described in these reports.

The reaction is, in general, conducted under an inert atmosphere such as the atmospheres of nitrogen, argon and helium under a normal pressure but it may also be conducted under a pressurized condition, where necessary. The reaction temperature is in the range of 15 to 300°C and preferably in the range of 30 to 200°C.

As the solvent for the reaction, water, aromatic hydrocarbons such as benzene, toluene and xylene, ethers such as 1,2-dimethoxyethane, diethyl ether, methyl t-butyl ether, tetrahydrofuran and dioxane; saturated hydrocarbons such as pentane, hexane, heptane, octane and cyclohexane, halides such as dichloromethane, chloroform, carbon tetrachloride, 1,2-dichloroethane and 1,1,1-trichloroethane, nitriles such as acetonitrile and benzonitrile, esters such as ethyl acetate, methyl acetate and butyl acetate, and amides such as N,N-dimethylformamide, N,N-dimethylacetamide and N-methylpyrrolidone, can be used singly or as a mixture. Among these solvents, toluene, 1,2-dimethoxyethane, dioxane and water are preferable. The amount by weight of the solvent is, in general, in the range of 3 to 50 times as much as and preferably in the range of 4 to 20 times as much as the amount by weight of the arylboronic acid or the derivative thereof.

Examples of the base used in the reaction include sodium carbonate, potassium carbonate, sodium hydroxide, potassium hydroxide, sodium hydrogencarbonate, potassium hydrogencarbonate, magnesium carbonate, lithium carbonate, potassium fluoride, cesium fluoride, cesium chloride, cesium bromide, cesium carbonate, potassium phosphate, methoxysodium, t-butoxypotassium, t-butoxysodium and t-butoxylithium. Among these bases, sodium carbonate is preferable. The amount of the base is, in general, in the range of 0.7 to 10 mole equivalents and preferably in the range of 0.9 to 6 mole equivalents based on the amount of the arylboronic acid or the derivative thereof.

Examples of the catalyst used in the reaction include palladium catalysts such as tetrakis(triphenylphosphine)palladium, dichlorobis-(triphenylphosphine)palladium, dichloro[bis(diphenylphosphino)ethane]-palladium, dichloro[bis(diphenylphosphino)propane]palladium, dichloro-[bis(diphenylphosphino)butane]palladium and dichloro[bis(diphenylphosphino)ferrocene]palladium; and nickel catalysts such as tetrakis-(triphenylphosphine)nickel, dichlorobis(triphenylphosphine)nickel, dichloro[bis(diphenylphosphino)ethane]nickel, dichloro[bis(diphenylphosphino)propane]nickel, dichloro[bis(diphenylphosphino)butane]nickel and dichloro[bis(diphenylphosphino)ferrocene]nickel. Among these catalysts, tetrakis(triphenylphosphine)palladium is preferable. The amount of the catalyst is, in general, in the range of 0.001 to 1 mole equivalent and preferably in the range of 0.01 to 0.1 mole equivalent based on the amount of the halogenated anthracene derivative.

Examples of the halogen atom in the halogen compound include iodine atom, bromine atom and chlorine atom. Iodine atom and bromine atom are preferable.

The boration reaction can be conducted in accordance with a known process (Jikken Kagaku Koza, 4th edition, edited by the Chemical Society of Japan, Volume 24, Pages 61 to 90; J. Org. Chem. Vol. 60, 7508 (1995); and others). For example, when the reaction contains the lithiation reaction or the Grignard reaction of a halogenated aryl compound, in general, the reaction is conducted under an inert atmosphere such as the atmospheres of nitrogen, argon and helium, and an inert solvent is used as the solvent. As the inert solvent, for example, a saturated hydrocarbon such as pentane, hexane, heptane, octane and cyclohexane, an ether such as 1,2-dimethoxyethane, diethyl ether, methyl t-butyl ether, tetrahydrofuran and dioxane, or an aromatic hydrocarbon such as toluene and xylene, can be used singly or as a mixed solvent. It is preferable that diethyl ether or toluene is used. The amount by weight of the solvent is, in general, in the range of 3 to 50 times as much as and preferably in the range of 4 to 20 times as much as the amount by weight of the halogenated aryl compound.

Examples of the lithiating agent include alkyl metal reagents such as n-butyllithium, t-butyllithium, phenyllithium and methyllithium; and amide bases such as lithium diisopropylamide and lithium bistrimethyl-silylamide. Among these agents, n-butyllithium is preferable. The Grignard reagent can be prepared by the reaction of the halogenated aryl compound and metallic magnesium. As the trialkyl borate, for example, trimethyl borate, triethyl borate, triisopropyl borate and tributyl borate can be used. Trimethyl borate and triisopropyl borate are preferable.

The amounts of the lithiating agent and the metallic magnesium are each, in general, in the range of 1 to 10 mole equivalents and preferably in the range of 1 to 2 mole equivalents base on the amount of the halogenated aryl compound. The amount of the trialkyl borate is, in general, in the range of 1 to 10 mole equivalents and preferably in the range of 1 to 5 mole equivalents based on the amount of the halogenated aryl compound. The reaction temperature is, in general, in the range of -100 to 50°C and preferably in the range of -75 to 10°C.

It is preferable that the bisanthracene derivative of the present invention is used as the light emitting material for organic EL devices and more preferably as the host material for organic EL devices.

The organic electroluminescence device of the present invention comprises a cathode, an anode and an organic thin film layer which comprises one layer or a plurality of layers comprising at least a light emitting layer and is disposed between the cathode and the anode, wherein the organic thin film layer comprises at least one compound selected from bisanthracene derivatives described above singly or as a component of a mixture.

In the organic EL device of the present invention, it is preferable that the light emitting layer further comprises an arylamine compound and/or a styrylamine compound.

As the styrylamine compound, compounds represented by the following general formula (A) are preferable: 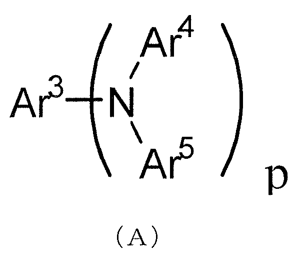 wherein Ar³ represent a group selected from phenyl group, biphenyl group, terphenyl group, stilbene group and distyrylaryl groups, Ar⁴ and Ar⁵ each represent hydrogen atom or an aromatic hydrocarbon group having 6 to 20 carbon atoms, the groups represented by Ar³, Ar⁴ and Ar⁵ may be substituted, p represents an integer of 1 to 4 and, preferably, at least one of the groups represented by Ar⁴ and Ar⁵ is substituted with styryl group.

Examples of the aromatic hydrocarbon group having 6 to 20 carbon atoms include phenyl group, naphthyl group, anthranyl group, phenanthryl group and terphenyl group.

As the arylamine compound, compounds represented by the following general formula (B) are preferable: 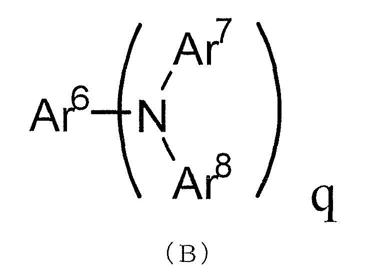 wherein Ar⁶ to Ar⁸ each represent a substituted or unsubstituted aryl group having 5 to 40 nuclear carbon atoms, and q represents an integer of 1 to 4.

Examples of the aryl group having 5 to 40 nuclear carbon atoms include phenyl group, naphthyl group, anthranyl group, phenanthryl group, pyrenyl group, coronyl group, biphenyl group, terphenyl group, pyrrolyl group, furanyl group, thiophenyl group, benzothiophenyl group, oxadiazolyl group, diphenylanthranyl group, indolyl group, carbazolyl group, pyridyl group, benzoquinolyl group, fluoranthenyl group, acenaphthofluoranthenyl group, stilbene group, perylenyl group, chrysenyl group, picenyl group, triphenylenyl group, rubicenyl group, benzoanthracenyl group, phenylanthranyl group, bisanthracenyl group and aryl groups represented by the following general formula (C) or expressed by the following formula (D). Among these groups, naphthyl group, anthranyl group, chrysenyl group, pyrenyl group and the aryl group expressed by formula (D) are preferable.

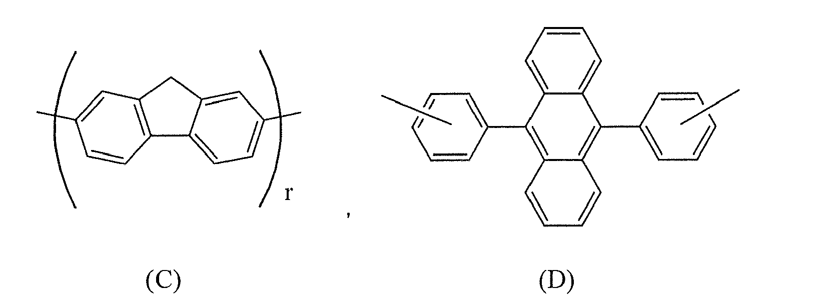

In general formula (C), **r** represents an integer of 1 to 3.

Preferable examples of the substituent to the aryl group include alkyl groups having 1 to 6 carbon atoms such as ethyl group, methyl group, i-propyl group, n-propyl group, s-butyl group, t-butyl group, pentyl group, hexyl group, cyclopentyl group and cyclohexyl group; alkoxyl groups having 1 to 6 carbon atoms such as ethoxyl group, methoxyl group, i-propoxyl group, n-propoxyl group, s-butoxyl group, t-butoxyl group, pentoxyl group, hexyloxyl group, cyclopentoxyl group and cyclohexyloxyl group; aryl groups having 5 to 40 nuclear carbon atoms; amino groups substituted with an aryl group having 5 to 40 nuclear carbon atoms; ester groups having an aryl group having 5 to 40 nuclear carbon atoms; ester groups having an alkyl group having 1 to 6 carbon atoms; cyano group; nitro group; and halogen atoms.

The construction of the organic EL device of the present invention will be described in the following.

Typical examples of the construction of the organic EL device include:
(1) An anode / a light emitting layer / a cathode;
(2) An anode / a hole injecting layer / a light emitting layer / a cathode;
(3) An anode / a light emitting layer / an electron injecting layer / a cathode;
(4) An anode / a hole injecting layer / a light emitting layer / an electron injecting layer / a cathode;
(5) An anode / an organic semiconductor layer / a light emitting layer / a cathode;
(6) An anode / an organic semiconductor layer / an electron barrier layer / a light emitting layer / a cathode;
(7) An anode / an organic semiconductor layer / a light emitting layer /an adhesion improving layer / a cathode;
(8) An anode / a hole injecting layer / a hole transporting layer / a light emitting layer / an electron injecting layer / a cathode;
(9) An anode / an insulating layer / a light emitting layer / an insulating layer / a cathode;
(10) An anode / an inorganic semiconductor layer / an insulating layer / a light emitting layer / an insulating layer / a cathode;
(11) An anode / an organic semiconductor layer / an insulating layer / a light emitting layer / an insulating layer / a cathode;
(12) An anode / an insulating layer / a hole injecting layer / a hole transporting layer / a light emitting layer / an insulating layer / a cathode; and
(13) An anode / an insulating layer / a hole injecting layer / a hole transporting layer / a light emitting layer / an electron injecting layer / a cathode.

Among the above constructions, construction (8) is preferable. However, the construction of the organic EL device is not limited to those shown above as the examples.

In the organic EL device of the present invention, it is preferable that the light emitting zone or the hole transporting zone comprises the bisanthracene derivative of the present invention among the constituting elements of the device although any of the organic layers may comprise the bisanthracene derivative. The content of the bisanthracene derivative is selected in the range of 30 to 100% by mole.

The organic EL device is, in general, prepared on a substrate transmitting light. The substrate transmitting light is the substrate supporting the organic EL device. It is preferable that the substrate transmitting light has a transmittance of light of 50% or greater in the visible region of 400 to 700 nm. It is also preferable that a flat and smooth substrate is used.

As the substrate transmitting light, for example, glass plates and synthetic resin plates are advantageously used. Examples of the glass plate include plates made of soda lime glass, glass containing barium and strontium, lead glass, aluminosilicate glass, borosilicate glass, barium borosilicate glass and quartz. Examples of the synthetic resin plate include plates made of polycarbonate resins, acrylic resins, polyethylene terephthalate resins, polyether sulfide resins and polysulfone resins.

The anode has the function of injecting holes into the hole transporting layer or the light emitting layer. It is effective that the anode has a work function of 4.5 eV or greater. Examples of the material for the anode used in the present invention include indium tin oxide alloys (ITO), tin oxide (NESA), gold, silver, platinum and copper. For the cathode, materials having a small work function are preferable for the purpose of injecting electrons into the electron transporting layer or the light emitting layer.

The anode can be prepared by forming a thin film of the electrode substance described above in accordance with a process such as the vapor deposition process and the sputtering process.

When the light emitted from the light emitting layer is obtained through the anode, it is preferable that the anode has a transmittance of the emitted light greater than 10%. It is also preferable that the sheet resistivity of the anode is several hundred Ω/□ or smaller. The thickness of the anode is, in general, selected in the range of 10 nm to 1 µm and preferably in the range of 10 to 200 nm although the range may be different depending on the used material.

The light emitting layer in the organic EL device of the present invention has the following functions:
(i) The injecting function: the function of injecting holes from the anode or the hole injecting layer and injecting electrons from the cathode or the electron injecting layer when an electric field is applied;
(ii) The transporting function: the function of transporting injected charges (electrons and holes) by the force of the electric field; and
(iii) The light emitting function : the function of providing the field for recombination of electrons and holes and leading the recombination to the emission of light.

As the process for forming the light emitting layer, a conventional process such as the vapor deposition process, the spin coating process and the LB process can be used. It is particularly preferable that the light emitting layer is a molecular deposit film. The molecular deposit film is a thin film formed by deposition of a material compound in the gas phase or a thin film formed by solidification of a material compound in a solution or in the liquid phase. In general, the molecular deposit film can be distinguished from the thin film formed in accordance with the LB process (the molecular accumulation film) based on the differences in aggregation structures and higher order structures and the functional differences caused by these structural differences.

As disclosed in Japanese Patent Application Laid-Open No. Showa 57(1982)-51781, the light emitting layer can also be formed by dissolving a binder such as a resin and the material compounds into a solvent to prepare a solution, followed by forming a thin film from the prepared solution in accordance with the spin coating process or the like.

In the present invention, where desired, the light emitting layer may comprise conventional light emitting materials other than the light emitting material comprising the bisanthracene derivative of the present invention, or a light emitting layer comprising other conventional light emitting material may be laminated to the light emitting layer comprising the light emitting material of the present invention as long as the object of the present invention is not adversely affected.

The hole injecting and transporting layer is a layer which helps injection of holes into the light emitting layer and transports the holes to the light emitting region. The layer exhibits a great mobility of holes and, in general, has an ionization energy as small as 5.5 eV or smaller. For the hole injecting and transporting layer, a material which transports holes to the light emitting layer under an electric field of a smaller strength is preferable. A material which exhibits, for example, a mobility of holes of at least 10-⁴ cm²/V·sec under application of an electric field of 10⁴ to 10⁶ V/cm is preferable. As the above material, a material can be selected as desired from materials which are conventionally used as the charge transporting material of holes in photoconductive materials and conventional materials which are used for the hole injecting layer in organic EL devices.

Examples include triazole derivatives (United States Patent No. 3,112,197), oxadiazole derivatives (United States Patent No. 3,189,447), imidazole derivatives (Japanese Patent Application Publication No. Showa 37(1962)-16096), polyarylalkane derivatives (United States Patent Nos. 3,615,402, 3,820,989 and 3,542,544; Japanese Patent Application Publication Nos. Showa 45(1970)-555 and Showa 51 (1976)-10983; and Japanese Patent Application Laid-Open Nos. Showa 51(1976)-93224, Showa 55(1980)-17105, Showa 56(1981)-4148, Showa 55(1980)-108667, Showa 55(1980)-156953 and Showa 56(1981)-36656); pyrazoline derivatives and pyrazolone derivatives (United States Patent Nos. 3,180,729 and 4,278,746; and Japanese Patent Application Laid-Open Nos. Showa 55(1980)-88064, Showa 55(1980)-88065, Showa 49(1974)-105537, Showa 55(1980)-51086, Showa 56(1981)-80051, Showa 56(1981)-88141, Showa 57(1982)-45545, Showa 54(1979)-112637 and Showa 55(1980)-74546); phenylenediamine derivatives (United Sates Patent No. 3,615,404; Japanese Patent Application Publication Nos. Showa 51(1976)-10105, Showa 46(1971)-3712 and Showa 47(1972)-25336; and Japanese Patent Application Laid-Open Nos. Showa 54(1979)-53435, Showa 54(1979)-110536 and Showa 54(1979)-119925); arylamine derivatives (United States Patent Nos. 3,567,450, 3,180,703, 3,240,597, 3,658,520, 4,232,103, 4,175,961 and 4,012,376; Japanese Patent Application Publication Nos. Showa 49(1974)-35702 and Showa 39(1964)-27577; Japanese Patent Application Laid-Open Nos. Showa 55(1980)-144250, Showa 56(1981)-119132 and Showa 56(1981)-22437; and West German Patent No. 1,110,518); chalcone derivatives substituted with amino group (United States Patent No. 3,526,501); oxazole derivatives (United States Patent No. 3,257,203); styrylanthracene derivatives (Japanese Patent Application Laid-Open Nos. Showa 56(1981)-46234); fluorenone derivatives (Japanese Patent Application Laid-Open Nos. Showa 54(1979)-110837); hydrazone derivatives (United States Patent No. 3,717,462; and Japanese Patent Application Laid-Open Nos. Showa 54(1979)-59143, Showa 55(1980)-52063, Showa 55(1980)-52064, Showa 55(1980)-46760, Showa 55(1980)-85495, Showa 57(1982)-11350, Showa 57(1982)-148749 and Heisei 2(1990)-311591); stilbene derivatives (Japanese Patent Application Laid-Open Nos. Showa 61(1986)-210363, Showa 61(1986)-228451, Showa 61(1986)-14642, Showa 61(1986)-72255, Showa 62(1987)-47646, Showa 62(1987)-36674, Showa 62(1987)-10652, Showa 62(1987)-30255, Showa 60(1985)-93455, Showa 60(1985)-94462, Showa 60(1985)-174749 and Showa 60(1985)-175052); silazane derivatives (United States Patent No. 4,950,950); polysilane-based compounds (Japanese Patent Application Laid-Open No. Heisei 2(1990)-204996); aniline-based copolymers (Japanese Patent Application Laid-Open No. Heisei 2(1990)-282263); and electrically conductive macromolecular oligomers (in particular, thiophene oligomers) disclosed in Japanese Patent Application Laid-Open No. Heisei 1(1989)-211399.

Besides the above materials which can be used as the material for the hole injecting layer, porphyrin compounds (compounds disclosed in Japanese Patent Application Laid-Open No. Showa 63(1988)-2956965); and aromatic tertiary amine compounds and styrylamine compounds (United States Patent No. 4,127,412 and Japanese Patent Application Laid-Open Nos. Showa 53(1978)-27033, Showa 54(1979)-58445, Showa 54(1979)-149634, Showa 54(1979)-64299, Showa 55(1980)-79450. Showa 55(1980)-144250, Showa 56(1981)-119132, Showa 61(1986)-295558, Showa 61(1986)-98353 and Showa 63(1988)-295695) are preferable, and the aromatic tertiary amines are more preferable.

Further examples include compounds having two condensed aromatic rings in the molecule which are described in the United States Patent No. 5,061,569 such as 4,4'-bis(N-(1-naphthyl)-N-phenylamino)-biphenyl (referred to as NPD, hereinafter) and a compound in which three triphenylamine units are bonded together in a star-burst shape, which is described in Japanese Patent Application Laid-Open No. Heisei 4(1992)-308688, such as 4,4',4"-tris(N-(3-methylphenyl)-N-phenylamino)-triphenylamine (referred to as MTDATA, hereinafter).

Besides the above bisanthracene derivatives, inorganic compounds such as Si of the p-type and SiC of the p-type can also be used as the material for the hole injecting layer.

The hole injecting and transporting layer can be formed by preparing a thin film of the above compound in accordance with a conventional process such as the vacuum vapor deposition process, the spin coating process, the casting process and the LB process. The thickness of the hole injecting and transporting layer is not particularly limited. In general, the thickness is 5 nm to 5 µm.

The organic semiconductor layer is a layer helping injection of holes or electrons into the light emitting layer. As the organic semiconductor layer, a layer having a conductivity of 10⁻¹⁰ S/cm or greater is preferable. As the material for the organic semiconductor layer, oligomers containing thiophene can be used, and conductive oligomers such as oligomers containing allylamine and conductive dendrimers such as dendrimers containing allylamine, which are disclosed in Japanese Patent Application Laid-Open No. Heisei 8(1996)-193191, can also be used.

The electron injecting and transporting layer is a layer which helps injection of electrons into the light emitting layer and transportation of the electrons to the light emitting region and exhibits a great mobility of electrons. The adhesion improving layer is an electron injecting layer comprising a material exhibiting improved adhesion with the cathode.

It is known that, in an organic EL device, emitted light is reflected at an electrode (the cathode in the present case), and the light emitted and obtained directly from the anode and the light obtained after reflection at the electrode interfere with each other. The thickness of the electron transporting layer is suitably selected in the range of several nm to several µm so that the interference is effectively utilized. When the thickness is great, it is preferable that the mobility of electrons is at least 10⁻⁵ cm²/Vs or greater under the application of an electric field of 10⁴ to 10⁶ V/cm so that the increase in the voltage is prevented.

As the material used for the electron injecting layer, metal complexes of 8-hydroxyquinoline and derivatives thereof and oxadiazole derivatives are preferable. Examples of 8-hydroxyquinoline and the derivative thereof include metal chelated oxinoid compounds including chelate compounds of oxines (in general, 8-quinolinol or 8-hydroxyquinoline). For example, tris(8-quinolinol)aluminum (Alq) can be used as the electron injecting material.

Examples of the oxadiazole derivative include electron transfer compounds represented by the following general formulae: 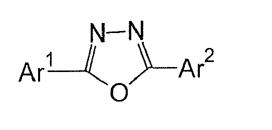 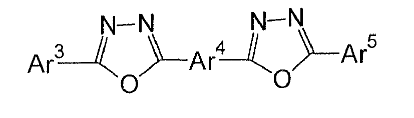 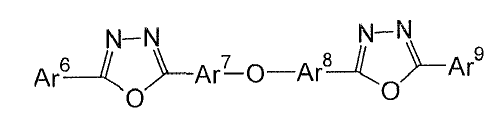 In the above formulae, Ar¹, Ar², Ar³, Ar⁵, Ar⁶ and Ar⁹ each represent a substituted or unsubstituted aryl group and may represent the same group or different groups. Ar⁴, Ar⁷ and Ar⁸ each represent a substituted or unsubstituted arylene group and may represent the same group or different groups.

Examples of the aryl group include phenyl group, biphenyl group, anthranyl group, perylenyl group and pyrenyl group. Examples of the arylene group include phenylene group, naphthylene group, biphenylene group, anthranylene group, perylenylene group and pyrenylene group. Examples of the substituent include alkyl groups having 1 to 10 carbon atoms, alkoxyl groups having 1 to 10 carbon atoms and cyano group. As the electron transfer compound, compounds which can form thin films are preferable.

Specific examples of the electron transfer compound include the following compounds:

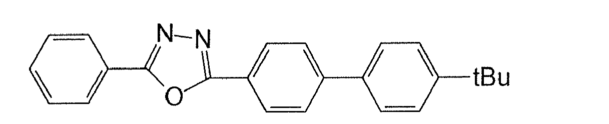

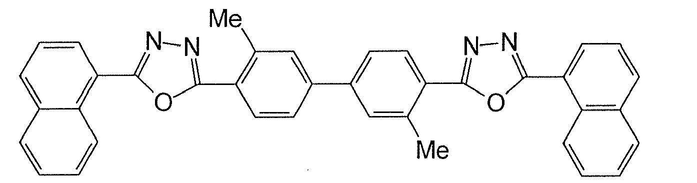

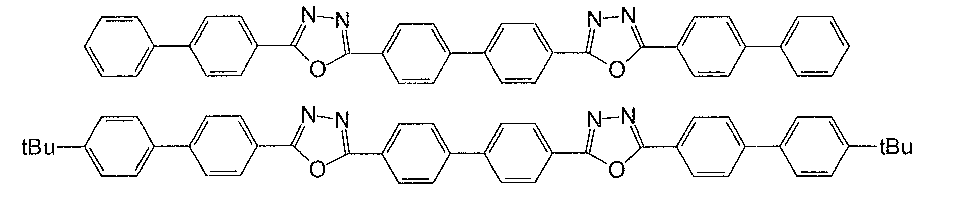

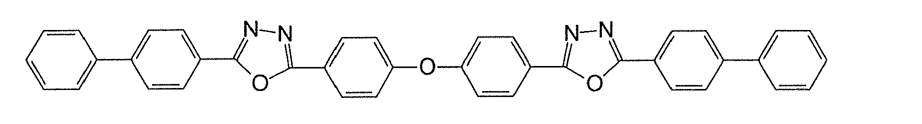

As the material which can be used for the electron injecting layer and the electron transporting layer, compounds represented by the following general formulae (E) to (J) can be used.

Heterocyclic derivatives having nitrogen atom represented by any one of general formulae (E) and (F):

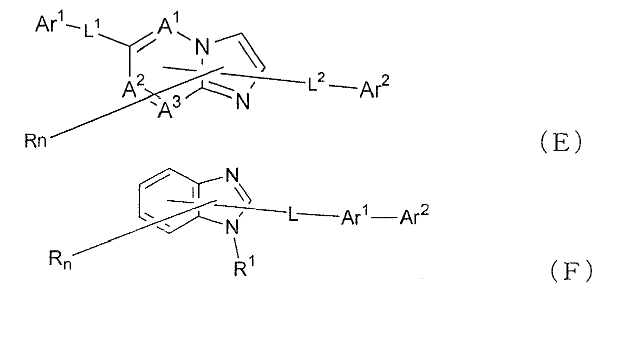

In general formulae (E) and (F), A¹ to A³ each independently represent nitrogen atom or carbon atom.

Ar¹ represents a substituted or unsubstituted aryl group having 6 to 60 nuclear carbon atoms or a substituted or unsubstituted heteroaryl group having 3 to 60 nuclear carbon atoms; Ar² represents hydrogen atom, a substituted or unsubstituted aryl group having 6 to 60 nuclear carbon atoms, a substituted or unsubstituted heteroaryl group having 3 to 60 nuclear carbon atoms, a substituted or unsubstituted alkyl group having 1 to 20 carbon atoms, a substituted or unsubstituted alkoxyl group having 1 to 20 carbon atoms or a divalent group derived from any of the above groups; and either one of Ar¹ and Ar² represents a substituted or unsubstituted condensed cyclic group having 10 to 60 nuclear carbon atoms or a substituted or unsubstituted monohetero condensed cyclic group having 3 to 60 nuclear carbon atoms.

L¹, L² and L each independently represent the single bond, a substituted or unsubstituted arylene group having 6 to 60 nuclear carbon atoms, a substituted or unsubstituted heteroarylene group having 3 to 60 nuclear carbon atoms or a substituted or unsubstituted fluorenylene group.

R represents hydrogen atom, a substituted or unsubstituted aryl group having 6 to 60 nuclear carbon atoms, a substituted or unsubstituted heteroaryl group having 3 to 60 nuclear carbon atoms, a substituted or unsubstituted alkyl group having 1 to 20 carbon atoms or a substituted or unsubstituted alkoxyl group having 1 to 20 carbon atoms; n represents an integer of 0 to 5; and, when n represents an integer of 2 or greater, the atoms and the groups represented by a plurality of R may be the same with or different from each other, and a plurality of groups represented by R which are adjacent to each other may be bonded to each other to form an aliphatic ring of the carbon ring type or an aromatic ring of the carbon ring type.

Heterocyclic derivatives having nitrogen atom represented by the following general formula (G):

HAr-L-Ar¹-Ar² (G)

In general formula (G), HAr represents a heterocyclic group having 3 to 40 carbon atoms and nitrogen atom which may have substituents, L represents the single bond, an arylene group having 6 to 60 carbon atoms which may have substituents, a heteroarylene group having 3 to 60 carbon atoms which may have substituents or a fluorenylene group which may have substituents, Ar¹ represents a divalent aromatic hydrocarbon group having 6 to 60 carbon atoms which may have substituents, and Ar² represents an aryl group having 6 to 60 carbon atoms which may have substituents or a heteroaryl group having 3 to 60 carbon atoms which may have substituents.

Silacyclopentadiene derivatives represented by the following general formula (H):

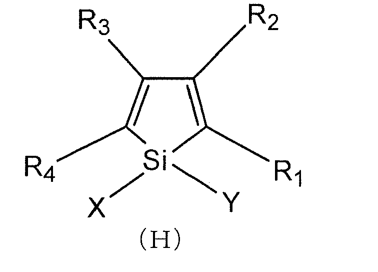

In general formula (H), X and Y each independently represent a saturated or unsaturated hydrocarbon group having 1 to 6 carbon atoms, an alkoxyl group, an alkenyloxyl group, an alkynyloxyl group, hydroxyl group, a substituted or unsubstituted aryl group, a substituted or unsubstituted heterocyclic group or a saturated or unsaturated cyclic group formed by bonding of the above groups represented by X and Y; and R₁ to R₄ each independently represent hydrogen atom, a halogen atom, a substituted or unsubstituted alkyl group having 1 to 6 carbon atoms, an alkoxyl group, an aryloxyl group, a perfluoroalkyl group, a perfluoroalkoxyl group, an amino group, an alkylcarbonyl group, an arylcarbonyl group, an alkoxycarbonyl group, an aryloxycarbonyl group, an azo group, an alkylcarbonyloxyl group, an arylcarbonyloxyl group, an alkoxycarbonyloxyl group, an aryloxycarbonyloxyl group, sulfinyl group, sulfonyl group, sulfanyl group, silyl group, carbamoyl group, an aryl group, a heterocyclic group, an alkenyl group, an alkynyl group, nitro group, formyl group, nitroso group, formyloxyl group, isocyano group, cyanate group, isocyanate group, thiocyanate group, isothiocyanate group, a cyano group or, when the groups are adjacent to each other, a structure formed by condensation of substituted or unsubstituted rings.

Borane derivatives represented by the following general formula (I):

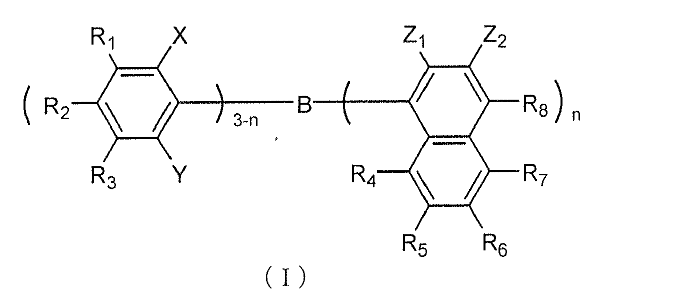

In general formula (I), R₁ to R₈ and Z₂ each independently represent hydrogen atom, a saturated or unsaturated hydrocarbon group, an aromatic hydrocarbon group, a heterocyclic group, a substituted amino group, a substituted boryl group, an alkoxyl group or an aryloxyl group; X, Y and Z₁ each independently represent a saturated or unsaturated hydrocarbon group, an aromatic hydrocarbon group, a heterocyclic group, a substituted amino group, an alkoxyl group or an aryloxyl group, and substituents to the groups represented by Z₁ and Z₂ may be bonded to each other to form a condensed ring; n represents an integer of 1 to 3 and, when n represents an integer of 2 or greater, a plurality of Z₁ may represent different groups; and the case where n represents 1, X, Y and R₂ each represent methyl group and R₈ represents hydrogen atom or a substituted boryl group and the case where n represents 3 and Z₁ represents methyl group are excluded.

Compounds represented by general formula (J):

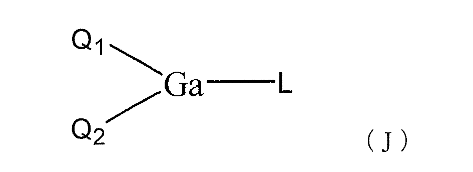

In general formula (J), Q¹ and Q² each independently represent a ligand represented by the following general formula (K): 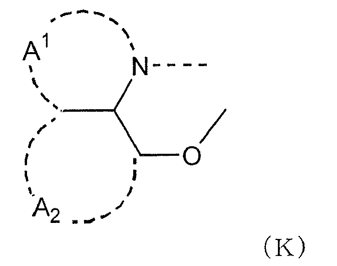 (rings A¹ and A² each representing six-membered aryl cyclic structure which may have substituents and are condensed with each other), L represents a halogen atom, a substituted or unsubstituted alkyl group, a substituted or unsubstituted cycloalkyl group, a substituted or unsubstituted aryl group, a substituted or unsubstituted heterocyclic group, -OR¹ (R¹ representing hydrogen atom, a substituted or unsubstituted alkyl group, a substituted or unsubstituted cycloalkyl group, a substituted or unsubstituted aryl group or a substituted or unsubstituted heterocyclic group) or -O-Ga-Q³(Q⁴) (Q³ and Q⁴ are as defined for Q¹ and Q²).

The above metal complex compound strongly exhibits the property as the n-type semiconductor and a great ability of electron injection. Since the energy of formation of the complex compound is small, the bonding between the metal and the ligand in the formed metal complex compound is strong, and the quantum efficiency of fluorescence as the light emitting material is great.

Examples of the substituent to rings A¹ ad A² forming the ligand represented by general formula (K) include halogen atoms such as chlorine atom, bromine atom, iodine atom and fluorine atom; substituted and unsubstituted alkyl groups such as methyl group, ethyl group, propyl group, butyl group, sec-butyl group, tert-butyl group, pentyl group, hexyl group, heptyl group, octyl group, stearyl group and trichloromethyl group; substituted and unsubstituted aryl groups such as phenyl group, naphthyl group, 3-methylphenyl group, 3-methoxyphenyl group, 3-fluorophenyl group, 3-trichloromethylphenyl group, 3-trifluoromethylphenyl group and 3-nitrophenyl group; substituted and unsubstituted alkoxyl groups such as methoxyl group, n-butoxyl group, tert-butoxyl group, trichloromethoxyl group, trifluoroethoxyl group, pentafluoropropoxyl group, 2,2,3,3-tetrafluoropropoxyl group, 1,1,1,3,3,3-hexafluoro-2-propoxyl group and 6-(perfluoroethyl)hexyloxyl group; substituted and unsubstituted aryloxyl groups such as phenoxyl group, p-nitrophenoxyl group, p-tert-butylphenoxyl group, 3-fluorophenoxyl group, pentafluorophenoxyl group and 3-triflurormethylphenoxyl group; substituted and unsubstituted alkylthio groups such as methylthio group, ethylthio group, tert-butylthio group, hexylthio group, octylthio group and trifluoromethylthio group; substituted and unsubstituted arylthio groups such as phenylthio group, p-nitrophenylthio group, p-tert-butylphenylthio group, 3-fluorophenylthio group, pentafluorophenylthio group and 3-trifluoromethylphenylthio group; cyano group; nitro group; amino group; mono- and disubstituted amino groups such as methylamino group, diethylamino group, ethylamino group, diethylamino group, dipropylamino group, dibutylamiono group and diphenylamino group; acylamino groups such as bis(acetoxymethyl)amino group, bis(acetoxyethyl)amino group, bis(acetoxypropyl)amino group and bis(acetoxybutyl)amino group; hydroxyl group; siloxyl group; acyl group; carbamoyl groups such as methylcarbamoyl group, dimethylcarbamoyl group, ethylcarbamoyl group, diethylcarbamoyl group, propylcarbamoyl group, butylcarbamoyl group and phenylcarbamoyl group; carboxylic acid group; sulfonic acid group; imide group; cycloalkyl groups such as cyclopentane group and cyclohexyl group; aryl groups such as phenyl group, naphthyl group, biphenyl group, anthranyl group, phenanthryl group, fluorenyl group and pyrenyl group; and heterocyclic groups such as pyridinyl group, pyrazinyl group, pyrimidinyl group, pyridazinyl group, triazinyl group, indolinyl group, quinolinyl group, acridinyl group, pyrrolidinyl group, dioxanyl group, piperidinyl group, morpholidinyl group, piperazinyl group, triatinyl group, carbazolyl group, furanyl group, thiophenyl group, oxazolyl group, oxadiazolyl group, benzoxazolyl group, thiazolyl group, thiadiazolyl group, benzothiazolyl group, triazolyl group, imidazolyl group, benzimidazolyl group and planyl group. The above substituents may be bonded to each other to form a six-membered aryl group or heterocyclic group.

A device comprising a reducing dopant in the interfacial region between a region transporting electrons or the cathode and the organic layer is preferable as an embodiment of the organic EL device of the present invention. The reducing dopant is defined as a substance which can reduce a compound having the electron transporting property. Various compounds can be used as the reducing dopant as long as the compounds have the specific reductive property. For example, at least one substance selected from the group consisting of alkali metals, alkaline earth metals, rare earth metals, oxides of alkali metals, halides of alkali metals, oxides of alkaline earth metals, halides of alkaline earth metals, oxides of rare earth metals, halides of rare earth metals, organic complexes of alkali metals, organic complexes of alkaline earth metals and organic complexes of rare earth metals can be advantageously used.

Preferable examples of the reducing dopant include substances having a work function of 2.9 eV or smaller, specific examples of which include at least one alkali metal selected from the group consisting of Na (the work function: 2.36 eV), K (the work function: 2.28 eV), Rb (the work function: 2.16 eV) and Cs (the work function: 1.95 eV) and at least one alkaline earth metal selected from the group consisting of Ca (the work function: 2.9 eV), Sr (the work function: 2.0 to 2.5 eV) and Ba (the work function: 2.52 eV). Among the above substances, at least one alkali metal selected from the group consisting of K, Rb and Cs is more preferable, Rb and Cs are still more preferable, and Cs is most preferable as the reducing dopant. These alkali metals have great reducing ability, and the luminance of the emitted light and the life of the organic EL device can be increased by addition of a relatively small amount of the alkali metal into the electron injecting zone. As the reducing dopant having a work function of 2.9 eV or smaller, combinations of two or more alkali metals are also preferable. Combinations having Cs such as the combinations of Cs and Na, Cs and K, Cs and Rb and Cs, Na and K are more preferable. The reducing ability can be efficiently exhibited by the combination having Cs. The luminance of emitted light and the life of the organic EL device can be increased by adding the combination having Cs into the electron injecting zone.

The organic EL device of the present invention may further comprise an electron injecting layer which is constituted with an insulating material or a semiconductor and disposed between the cathode and the organic layer. By the electron injecting layer, leak of electric current can be effectively prevented, and the electron injecting property can be improved. As the insulating material, at least one metal compound selected from the group consisting of alkali metal chalcogenides, alkaline earth metal chalcogenides, halides of alkali metals and halides of alkaline earth metals is preferable. It is preferable that the electron injecting layer is constituted with the above substance such as the alkali metal chalcogenide since the electron injecting property can be further improved. Preferable examples of the alkali metal chalcogenide include Li₂O, LiO, Na₂S, Na₂Se and NaO. Preferable examples of the alkaline earth metal chalcogenide include CaO, BaO, SrO, BeO, BaS and CaSe. Preferable examples of the halide of an alkali metal include LiF, NaF, KF, LiCl, KCl and NaCl. Preferable examples of the halide of an alkaline earth metal include fluorides such as CaF₂, BaF₂, SrF₂, MgF₂ and BeF₂ and halides other than the fluorides.

Examples of the semiconductor constituting the electron transporting layer include oxides, nitrides and oxide nitrides of at least one metal selected from Ba, Ca, Sr, Yb, Al, Ga, In, Li, Na, Cd, Mg, Si, Ta, Sb and Zn used singly or in combination of two or more. It is preferable that the inorganic compound constituting the electron transporting layer forms a crystallite or amorphous insulating thin film. When the electron injecting layer is constituted with the insulating thin film described above, a more uniform thin film can be formed, and defects of pixels such as dark spots can be decreased. Examples of the inorganic compound include alkali metal chalcogenides, alkaline earth metal chalcogenides, halides of alkali metals and halides of alkaline earth metals which are described above.

For the cathode, a material such as a metal, an alloy, a conductive compound or a mixture of these materials which has a small work function (4 eV or smaller) is used as the electrode material. Examples of the electrode material include sodium, sodium-potassium alloys, magnesium, lithium, magnesium-silver alloys, aluminum/aluminum oxide, Al/Li₂O, Al/LiO₂, Al/LiF, aluminum-lithium alloys, indium and rare earth metals.

The cathode can be prepared by forming a thin film of the electrode material described above in accordance with a process such as the vapor deposition process and the sputtering process.

When the light emitted from the light emitting layer is obtained through the cathode, it is preferable that the cathode has a transmittance of the emitted light greater than 10 %. It is also preferable that the sheet resistivity of the cathode is several hundred Ω/□ or smaller. The thickness of the cathode is, in general, selected in the range of 10 nm to 1 µm and preferably in the range of 50 to 200 nm.

Defects in pixels tend to be formed in organic EL device due to leak and short circuit since an electric field is applied to ultra-thin films. To prevent the formation of the defects, a layer of a thin film having an insulating property may be inserted between the pair of electrodes.

Examples of the material used for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide and vanadium oxide. Mixtures and laminates of the above compounds can also be used.

To prepare the organic EL device of the present invention, for example, the anode, the light emitting layer and, where necessary, the hole injecting layer and the electron injecting layer are formed in accordance with the above process using the above materials, and the cathode is formed in the last step. The organic EL device may be prepared by forming the above layers in the order reverse to that described above, i.e., the cathode being formed in the first step and the anode in the last step.

An embodiment of the process for preparing an organic EL device having a construction in which an anode, a hole injecting layer, a light emitting layer, an electron injecting layer and a cathode are disposed successively on a substrate transmitting light will be described in the following.

On a suitable substrate which transmits light, a thin film made of a material for the anode is formed in accordance with the vapor deposition process or the sputtering process so that the thickness of the formed thin film is 1 µm or smaller and preferably in the range of 10 to 200 nm. The formed thin film is used as the anode. Then, a hole injecting layer is formed on the anode. The hole injecting layer can be formed in accordance with the vacuum vapor deposition process, the spin coating process, the casting process or the LB process, as described above. The vacuum vapor deposition process is preferable since a uniform film can be easily obtained and the possibility of formation of pin holes is small. When the hole injecting layer is formed in accordance with the vacuum vapor deposition process, in general, it is preferable that the conditions are suitably selected in the following ranges: the temperature of the source of the deposition: 50 to 450°C; the vacuum: 10⁻⁷ to 10⁻³ Torr; the rate of deposition: 0.01 to 50 nm/second; the temperature of the substrate: -50 to 300°C and the thickness of the film: 5 nm to 5 µm; although the conditions of the vacuum vapor deposition are different depending on the used compound (the material for the hole injecting layer) and the crystal structure and the recombination structure of the hole injecting layer to be formed.

Then, the light emitting layer is formed on the hole injecting layer formed above. Using a desired organic light emitting material, a thin film of the organic light emitting material can be formed in accordance with the vacuum vapor deposition process, the sputtering process, the spin coating process or the casting process, and the formed thin film is used as the light emitting layer. The vacuum vapor deposition process is preferable since a uniform film can be easily obtained and the possibility of formation of pin holes is small. When the light emitting layer is formed in accordance with the vacuum vapor deposition process, in general, the conditions of the vacuum vapor deposition process can be selected in the same ranges as those described for the vacuum vapor deposition of the hole injecting layer although the conditions are different depending on the used compound. It is preferable that the thickness is in the range of 10 to 40 nm.

The electron injecting layer is formed on the light emitting layer formed above. Similarly to the hole injecting layer and the light emitting layer, it is preferable that the electron injecting layer is formed in accordance with the vacuum vapor deposition process since a uniform film must be obtained. The conditions of the vacuum vapor deposition can be selected in the same ranges as those described for the vacuum vapor deposition of the hole injecting layer and the light emitting layer.

The cathode is formed on the electron injecting layer formed above in the last step, and the organic EL device can be obtained. The cathode is made of a metal and can be formed in accordance with the vacuum vapor deposition process or the sputtering process. It is preferable that the vacuum vapor deposition process is used in order to prevent formation of damages on the lower organic layers during the formation of the film.

In the above preparation of the organic EL device, it is preferable that the above layers from the anode to the cathode are formed successively while the preparation system is kept in a vacuum after being evacuated once.

The process for forming the layers in the organic EL device of the present invention is not particularly limited. A conventional process such as the vacuum vapor deposition process and the spin coating process can be used. The organic thin film layer which is used in the organic EL device of the present invention and comprises the compound represented by general formula (1) described above can be formed in accordance with a conventional process such as the vacuum vapor deposition process and the molecular beam epitaxy process (the MBE process) or, using a solution prepared by dissolving the compounds into a solvent, in accordance with a coating process such as the dipping process, the spin coating process, the casting process, the bar coating process and the roll coating process.

The thickness of each layer in the organic thin film layer in the organic EL device of the present invention is not particularly limited. A thickness in the range of several nanometers to 1 µm is preferable so that defects such as pin holes are decreased and the efficiency can be improved.

When a direct voltage is applied to the organic EL device, emission of light can be observed under application of a voltage of 5 to 40 V in the condition that the anode is connected to a positive electrode (+) and the cathode is connected to a negative electrode (-). When the connection is reversed, no electric current is observed and no light is emitted at all. When an alternating voltage is applied to the organic EL device, the uniform light emission is observed only in the condition that the polarity of the anode is positive and the polarity of the cathode is negative. When an alternating voltage is applied to the organic EL device, any type of wave shape can be used.

### EXAMPLES

The present invention will be described more specifically with reference to examples in the following. However, the present invention is not limited to the examples.

### Synthesis Example 1 (Synthesis of Compound AN-3)

Commercial m-bromoiodobenzene in an amount of 28 g, 20 g of commercial 3-bromophenylboronic acid and 300 ml of toluene were mixed. To the resultant mixture, 5.7 g of tetrakis-triphenylphosphinepalladium and 200 ml of a 2M aqueous solution of sodium carbonate were added, and the reactor was purged with argon. The obtained mixture was heated under the refluxing condition for 6 hours, cooled by being left standing and subjected to extraction with toluene. The organic layer was washed with water and a saturated aqueous solution of sodium chloride and then dried with anhydrous sodium sulfate. After the solvent was removed by distillation using an evaporator, the residue was purified in accordance with the silica gel column chromatography (the solvent for development: hexane), and 21.9 g of 3,3'-dibromobiphenyl was obtained as a white solid substance (the yield: 71%).

3,3'-Dibromobiphenyl obtained above in an amount of 6.2 g, 14.3 g of 10-phenylanthracene-9-boronic acid synthesized in accordance with a conventional process and 65 ml of dimethoxyethane (DME) were mixed together. To the resultant mixture, 1.2 g of tetrakistriphenylphosphinepalladium and 40 ml of a 2M aqueous solution of sodium carbonate were added, and the reactor was purged with argon. The obtained mixture was heated under the refluxing condition for 8 hours and cooled by being left standing, and the formed crystals were separated by filtration. After being washed with water and methanol, the crystals were washed with heated toluene, and 9.3 g of the object compound (Compound AN-3) was obtained as a white solid substance (the yield: 71%).

The obtained compound was examined in accordance with FD-MS (the field desorption mass analysis). Since m/z=658 corresponding to C₅₂H₃₄=658, the obtained compound was identified to be Compound AN-3.

### Synthesis Example 2 (Synthesis of Compound AN-4)

Commercial p-iodoaniline in an amount of 12.5 g, 12.6 g of commercial 3-bromophenylboronic acid and 180 ml of toluene were mixed. To the resultant mixture, 2.0 g of tetrakistriphenylphosphinepalladium and 110 ml of a 2M aqueous solution of sodium carbonate were added, and the reactor was purged with argon. The obtained mixture was heated under the refluxing condition for 8 hours, cooled by being left standing and subjected to extraction with toluene. The organic layer was washed with water and a saturated aqueous solution of sodium chloride and then dried with anhydrous sodium sulfate. After the solvent was removed by distillation using an evaporator, the residue was purified in accordance with the silica gel column chromatography (the solvent for development: ethyl acetate/hexane=1/4), and 7.9 g of 4-(3-bromophenyl)aniline was obtained as a brown solid substance (the yield: 56%).

Water in an amount of 20 ml and 30 ml of a concentrated hydrochloric acid were mixed, and 7.5 g of 4-(3-bromophenyl)aniline obtained above was added to the resultant mixture. The obtained mixture was cooled with ice, and an aqueous solution of sodium nitrite (NaNO₂ 2.3 g; water 10 ml) was added dropwise. After the resultant mixture was stirred for 1 hour, the reaction fluid was added dropwise to an aqueous solution of potassium iodide (KI 45 g; water 120 ml) at the room temperature. After the resultant fluid was stirred for 4 hours at the room temperature, 100 ml of chloroform and 1.3 g of sodium hydrogensulfite were added. The reaction fluid was subjected to extraction with chloroform, and the organic layer was washed with a 10% aqueous solution of sodium hydrogensulfite, water and a saturated solution of sodium chloride and dried with anhydrous sodium sulfate. After the solvent was removed by distillation using an evaporator, the residue was purified in accordance with the silica gel column chromatography (the solvent for development : hexane), and 7.5 g of 3-bromo-4'-iodobiphenyl was obtained as a white solid substance (the yield: 69%).

3-Bromo-4'-iodobiphenyl obtained above in an amount of 5.2 g, 10.4 g of 10-phenylanthracene-9-boronic acid synthesized in accordance with a conventional process and 50 ml of DME were mixed together. To the resultant mixture, 0.84 g of tetrakistriphenylphosphinepalladium and 30 ml of a 2M aqueous solution of sodium carbonate were added, and the reactor was purged with argon. The obtained mixture was heated under the refluxing condition for 8.5 hours and cooled by being left standing, and the formed crystals were separated by filtration. After being washed with water and methanol, the crystals were washed with methylene chloride, and 4.2 g of the object compound (Compound AN-4) was obtained as a light yellow solid substance (the yield: 44%).

The obtained compound was examined in accordance with FD-MS. Since m/z=658 corresponding to C₅₂H₃₄=658, the obtained compound was identified to be Compound AN-4.

### Synthesis Example 3 (Synthesis of Compound A-6)

In accordance with the same procedures as those conducted in Synthesis Example 1 except that 10-(naphthalen-2-yl)anthracen-9-boronic acid was used in place of 10-phenylanthracene-9-boronic acid, the object compound (Compound AN-6) was obtained as a grayish yellow solid substance (the yield: 85%).

The obtained compound was examined in accordance with FD-MS. Since m/z=758 corresponding to C₆₀H₃₈=758, the obtained compound was identified to be Compound AN-6.

### Synthesis Example 4 (Synthesis of Compound AN-10)

Commercial 1,3-diiodobenzene in an amount of 9.9 g, 14.5 g of commercial 3-bromophenylboronic acid and 100 ml of toluene were mixed. To the resultant mixture, 1.7 g of tetrakistriphenylphosphinepalladium and 60 ml of a 2M aqueous solution of sodium carbonate were added, and the reactor was purged with argon. The obtained mixture was heated under the refluxing condition for 7.5 hours, cooled by being left standing and subjected to extraction with toluene. The organic layer was washed with water and a saturated aqueous solution of sodium chloride and then dried with anhydrous sodium sulfate. After the solvent was removed by distillation using an evaporator, the residue was purified in accordance with the silica gel column chromatography (the solvent for development: hexane/toluene=19/1), and 5.8 g of 3,3"-dibromo-1,1',3',1"-terphenyl was obtained as a colorless oil (the yield: 50%).

3,3"-Dibromo-1,1',3',1"-terphenyl obtained above in an amount of 5.8 g, 10.7 g of 10-phenylanthracene-9-boronic acid synthesized in accordance with a conventional process and 100 ml of DME were mixed together. To the resultant mixture, 0.87 g of tetrakistriphenylphosphine palladium and 60 ml of a 2M aqueous solution of sodium carbonate were added, and the reactor was purged with argon. The obtained mixture was heated under the refluxing condition for 8 hours and cooled by being left standing, and the formed crystals were separated by filtration. The crystals were washed with water and methanol and then recrystallized from toluene, and 6.0 g of the object compound (Compound AN-10) was obtained as a light yellow solid substance (the yield: 54%).

The obtained compound was examined in accordance with FD-MS. Since m/z=734 corresponding to C₅₈H₃₈=734, the obtained compound was identified to be Compound AN-10.

### Synthesis Example 5 (Synthesis of Compound AN-15)

10-Phenylanthracene-9-boronic acid synthesized in accordance with a conventional process in an amount of 4.6 g, 5.6 g of 3-bromo-4'-iodo-biphenyl obtained in Synthesis Example 2 and 100 ml of toluene were mixed. To the resultant mixture, 0.54 g of tetrakistriphenylphosphinepalladium and 27 ml of a 2M aqueous solution of sodium carbonate were added, and the reactor was purged with argon. The obtained mixture was heated under the refluxing condition for 8.5 hours and cooled by being left standing, and the formed crystals were separated by filtration. The crystals were washed with water and methanol and then purified in accordance with the silica gel column chromatography (the solvent for development: toluene), and 4.9 g of 9-(3'-bromobiphenyl-4-yl)-10-phenyl-anthracene was obtained as a light yellow solid substance (the yield: 65%).

9-(3'-Bromobiphenyl-4-yl)-10-phenylanthracene obtained above in an amount of 4.9 g, 3.9 g of 10-(naphthalen-2-yl)anthracene-9-boronic acid synthesized in accordance with a conventional process and 80 ml of DME were mixed together. To the resultant mixture, 0.35 g of tetrakis-triphenylphosphinepalladium and 18 ml of a 2M aqueous solution of sodium carbonate were added, and the reactor was purged with argon. The obtained mixture was heated under the refluxing condition for 8 hours and cooled by being left standing, and the formed crystals were separated by filtration. After being washed with water and methanol, the crystals were washed with heated toluene, and 5.1 g of the object compound (Compound AN-15) was obtained as a light yellow solid substance (the yield: 72%).

The obtained compound was examined in accordance with FD-MS. Since m/z=708 corresponding to C₅₆H₃₆=708, the obtained compound was identified to be Compound AN-15.

### Synthesis Example 6 (Synthesis of Compound AN-28)

Commercial 2,6-dibromonaphthalene in an amount of 1.5 g, 4.1 g of 3-(9-phenylanthracen-10-yl)phenylboronic acid synthesized in accordance with a conventional process, 35 ml of toluene and 40 ml of DME were mixed together. To the resultant mixture, 0.49 g of tetrakistriphenylphosphinepalladium and 25 ml of a 2M aqueous solution of sodium carbonate were added, and the reactor was purged with argon. The obtained mixture was heated under the refluxing condition for 8 hours and cooled by being left standing, and the formed crystals were separated by filtration. After being washed with water and methanol, the crystals were washed with heated toluene, and 2.7 g of the object compound (Compound AN-28) was obtained as a white solid substance (the yield: 65%).

The obtained compound was examined in accordance with FD-MS. Since m/z=784 corresponding to C₆₂H₄₀=784, the obtained compound was identified to be Compound AN-28.

### Synthesis Example 7 (Synthesis of Compound AN-37)

In accordance with the same procedures as those conducted in Synthesis Example 6 except that 4,4'-diiodobiphenyl was used in place of 2,6-dibromonaphthalene, the object compound (Compound AN-37) was obtained as a grayish yellow solid substance (the yield: 81%).

The obtained compound was examined in accordance with FD-MS. Since m/z=810 corresponding to C₆₄H₄₂=810, the obtained compound was identified to be Compound AN-37.

### Synthesis Example 8 (Synthesis of Compound AN-39)

In accordance with the same procedures as those conducted in Synthesis Example 6 except that 2,7-diiodo-9,9-dimethyl-9H-fluorene was used in place of 2,6-dibromonaphthalene, the object compound (Compound AN-39) was obtained as a light yellow solid substance (the yield: 73%).

The obtained compound was examined in accordance with FD-MS. Since m/z=850 corresponding to C₆₇H₄₆=850, the obtained compound was identified to be Compound AN-39.

### Example 1 (Preparation of an organic EL device)

A glass substrate (manufactured by GEOMATEC Company) of 25 mm × 75 mm × 1.1 mm thickness having an ITO transparent electrode was cleaned by application of ultrasonic wave in isopropyl alcohol for 5 minutes and then by exposure to ozone generated by ultraviolet light for 30 minutes. The cleaned glass substrate having the transparent electrode was attached to a substrate holder of a vacuum vapor deposition apparatus. On the surface of the cleaned substrate at the side having the transparent electrode, a film of N,N'-bis(N,N'-diphenyl-4-aminophenyl)-N,N-diphenyl-4,4'-diamino-1,1'-biphenyl shown below (referred to as "TPD232 film", hereinafter) having a thickness of 60 nm was formed in a manner such that the formed film covered the transparent electrode. The formed TPD232 film worked as the hole injecting layer. On the formed TPD232 film, a film of N,N,N',N'-tetra(4-biphenyl)diamino-biphenylene shown below (referred to as "TBDB film", hereinafter) having a thickness of 20 nm was formed. The formed TBDB film worked as the hole transporting layer. On the formed TBDB film, Compound AN-3 prepared above was vapor deposited as the host material to form a film having a thickness of 40 nm. At the same time, an amine compound having styryl group (BD1) shown below as the light emitting material was vapor deposited in an amount such that the ratio of the amounts by weight of AN-3 to BD1 were 40:2. The formed film worked as the light emitting layer. On the formed film, a film of Alq shown below having a thickness of 10 nm was formed. This film worked as the electron injecting layer. On the film formed above, Li (the source of lithium: manufactured by SAES GETTERS Company) as the reducing dopant and Alq shown below were binary vapor deposited, and an Alq:Li film (the thickness: 10 nm) was formed as the electron injecting layer (or the cathode). On the formed Alq:Li film, metallic aluminum was vapor deposited to form a metal cathode, and an organic EL device was prepared.

The obtained organic EL device was examined by passing electric current. Blue light was emitted at a luminance of emitted light of 670 cd/m² under a voltage of 6.8 V and a current density of 10 mA/cm². The initial luminance was set at 1,000 cd/m², and the half life of the obtained organic EL device was measured. The efficiency of light emission in the high luminance region (30,000 cd/m²) was also measured. The results are shown in Table 1.

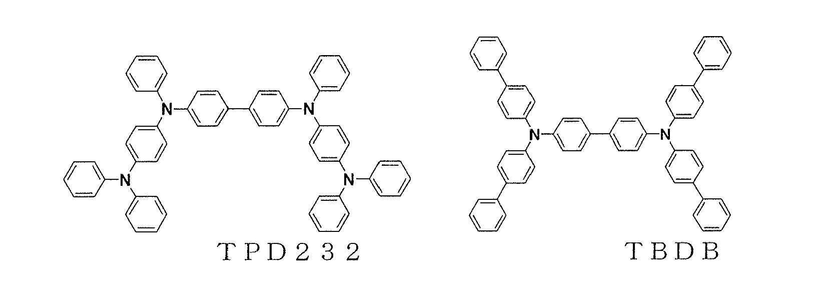

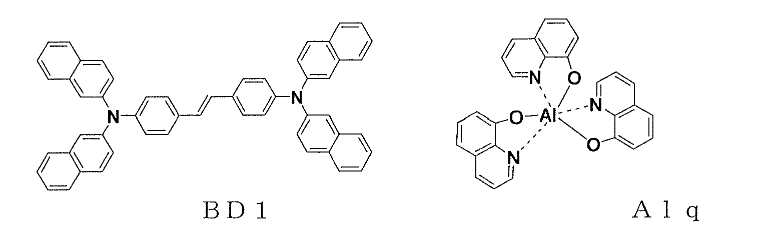

### Examples 2 to 8 (Preparation of organic EL devices)

Organic EL devices were prepared in accordance with the same procedures as those conducted in Example 1 except that compounds shown in Table 1 were used as the material for the light emitting layer in place of Compound AN-3.

The initial luminance was set at 1,000 cd/m², and the half life of the obtained organic EL devices was measured. The efficiency of light emission in the high luminance region (30,000 cd/m²) was also measured. The results are shown in Table 1.

### Example 9

An organic EL device was prepared in accordance with the same procedures as those conducted in Example 1 except that Compound AN-10 was used as the material for the light emitting layer in place of Compound AN-3 and amine compound BD2 shown below was used in place of amine compound BD1.

The initial luminance was set at 1,000 cd/m², and the half life of the obtained organic EL device was measured. The efficiency of light emission in the high luminance region (30,000 cd/m²) was also measured. The results are shown in Table 1.

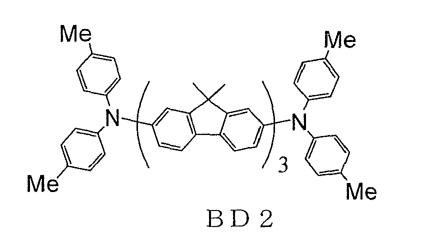

In the above formula, Me represents methyl group.

### Comparative Examples 1 to 6

Organic EL devices were prepared in accordance with the same procedures as those conducted in Example 1 except that an-1 (Comparative Example 1), an-2 (Comparative Example 2), an-3 (Comparative Example 3), an-4 (Comparative Example 4), an-5 (Comparative Example 5) or an-6 (Comparative Example 6) shown below was used as the material for the light emitting layer in place of Compound AN-3.

The initial luminance was set at 1,000 cd/m², and the half life of the obtained organic EL device was measured. The efficiency of light emission in the high luminance region (30,000 cd/m²) was also measured. The results are shown in Table 1.

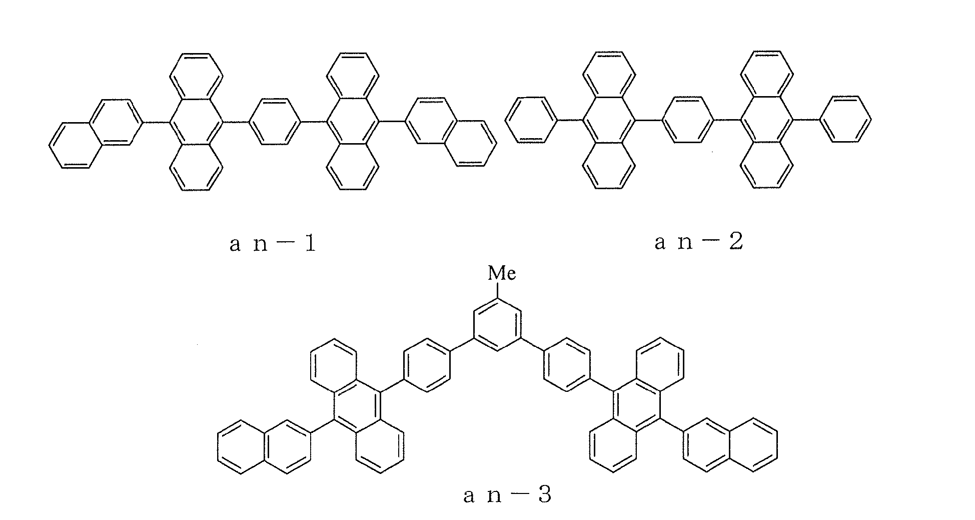

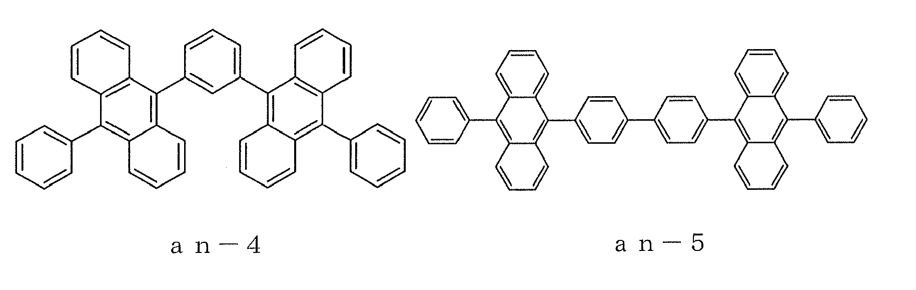

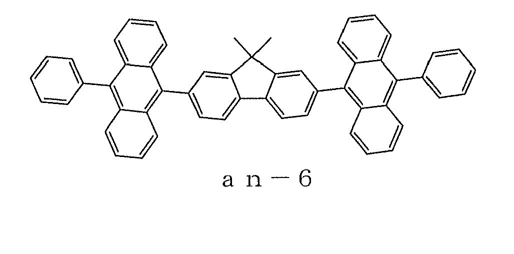

**Table 1**

| | Compound of light emitting layer | Half life (hour) | Efficiency of light emission in high luminance region (30,000 cd/m²) (cd/A) |
|---|---|---|---|
| Example 1 | AN-3/BD1 | 5200 | 5.00 |
| Example 2 | AN-4/BD1 | 6000 | 4.71 |
| Example 3 | AN-6/BD1 | 5700 | 4.72 |
| Example 4 | AN-10/BD1 | 6400 | 5.50 |
| Example 5 | AN-15/BD1 | 6200 | 4.75 |
| Example 6 | AN-28/BD1 | 6000 | 4.73 |
| Example 7 | AN-37/BD1 | 5900 | 4.71 |
| Example 8 | AN-39/BD1 | 4900 | 4.70 |
| Example 9 | AN-10/BD2 | 4800 | 5.13 |
| Comparative Example 1 | an-1/BD1 | 1900 | 4.16 |
| Comparative Example 2 | an-2/BD1 | 3200 | 4.49 |
| Comparative Example 3 | an-3/BD1 | 1500 | 4.30 |
| Comparative Example 4 | an-4/BD1 | 3400 | 4.47 |
| Comparative Example 5 | an-5/BD1 | 2800 | 4.20 |
| Comparative Example 6 | an-6/BD1 | 1800 | 4.28 |

As shown in Table 1, the organic EL devices prepared in Examples 1 to 9 using the compound of the present invention having the structure represented by general formula (1) exhibited greater efficiencies of light emission in the high luminance region and had longer lives than those of the organic EL devices prepared in Comparative Examples 1 to 6 using compounds not satisfying the requirement of the present invention.

### Example 10

A glass substrate (manufactured by GEOMATEC Company) of 25 mm × 75 mm × 1.1 mm thickness having an ITO transparent electrode having a thickness of 120 nm was cleaned by application of ultrasonic wave in isopropyl alcohol for 5 minutes and then by exposure to ozone generated by ultraviolet light for 30 minutes. The cleaned glass substrate having the transparent electrode was attached to a substrate holder of a vacuum vapor deposition apparatus. On the surface of the cleaned substrate at the side having the transparent electrode, TPD232 film having a thickness of 60 nm was formed in a manner such that the formed film covered the transparent electrode. The formed TPD232 film worked as the hole injecting layer. On the formed TPD232 film, TBDB film having a thickness of 20 nm was formed. The formed TBDB film worked as the hole transporting layer. On the formed TBDB film, Compound AN-4 prepared above was vapor deposited as the host material to form a film having a thickness of 40 nm. At the same time, a compound (GD1) shown below was vapor deposited as the dopant material in an amount such that the ratio of the amounts by weight of AN-4 to GD1 were 40:3. The formed film worked as the light emitting layer. On the formed film, a film of Alq having a thickness of 20 nm was formed. This film worked as the electron injecting layer. On the film formed above, LiF (the source of LiF: manufactured by SAES GETTERS Company) as the reducing dopant was vapor deposited to form a layer having a thickness of 1 nm, and then aluminum was vapor deposited to form a film having a thickness of 150 nm. The formed LiF/Al film worked as the cathode. An organic EL device was prepared in this manner.

The obtained organic EL device was examined by passing electric current. Green light (the maximum wavelength of emitted light: 533 nm) having a luminance of emitted light of 2,437 cd/m² was emitted at an efficiency of light emission of 24.7 cd/A under a voltage of 6.5 V and a current density of 10 mA/cm². The initial luminance was set at 3,000 cd/m², and the direct electric current was passed. The half life of the obtained organic EL device was 7,590 hours.

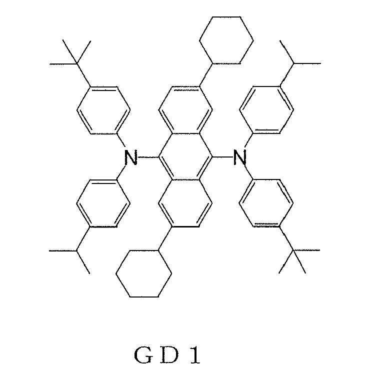

### Example 11

An organic EL device was prepared in accordance with the same procedures as those conducted in Example 10 except that Compound AN-10 was used as the material for the light emitting layer in place of Compound AN-4.

The obtained organic EL device was examined by passing electric current. Green light (the maximum wavelength of emitted light: 530 nm) having a luminance of emitted light of 2,485 cd/m² was emitted at an efficiency of light emission of 25.5 cd/A under a voltage of 6.4 V and a current density of 10 mA/cm². The initial luminance was set at 3,000 cd/m², and the direct electric current was passed. The half life of the obtained organic EL device was 8,530 hours.

### Comparative Example 7

An organic EL device was prepared in accordance with the same procedures as those conducted in Example 10 except that Compound an-2 was used as the material for the light emitting layer in place of Compound AN-4.

The obtained organic EL device was examined by passing electric current. Green light (the maximum wavelength of emitted light: 535 nm) having a luminance of emitted light of 1,228 cd/m² was emitted at an efficiency of light emission of 9.5 cd/A under a voltage of 7.0 V and a current density of 10 mA/cm². The initial luminance was set at 3,000 cd/m², and the direct electric current was passed. The half life of the obtained organic EL device was 1,930 hours.

As shown in the above, the organic EL devices prepared in Examples 10 and 11 using the compound of the present invention having the structure represented by general formula (1) exhibited greater luminances of emitted light and efficiencies of light emission at lower voltages and had longer lives than those of the organic EL devices prepared in Comparative Example 7 using compounds not satisfying the requirement of the present invention.

### INDUSTRIAL APPLICABILITY

As described specifically, the organic EL device comprising the bisanthracene derivative of the present invention exhibits a great efficiency of light emission in the high luminance region and has a long life. Therefore, the organic El device is remarkably useful as the organic EL device which is expected to be used continuously for a long time.

## Claims

1. A bisanthracene derivative represented by following general formula (1): 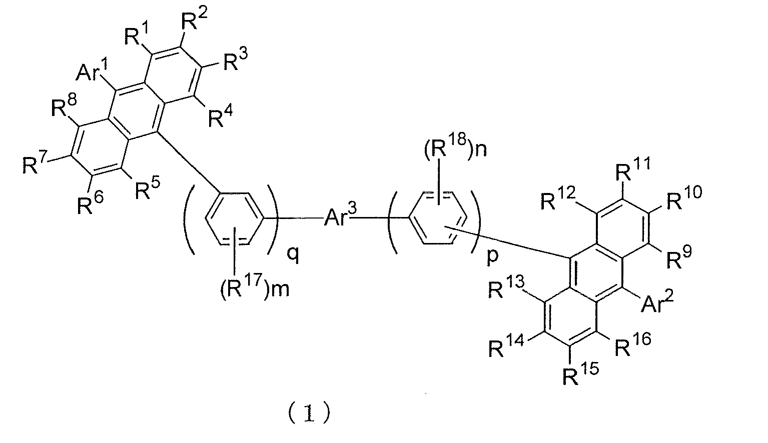 wherein Ar¹ and Ar² each independently represent a substituted or unsubstituted aromatic hydrocarbon group having 6 to 50 nuclear carbon atoms, and Ar³ represents a substituted or unsubstituted phenylene group, naphthylene group, chrysenyl group, biphenylene group or fluorenylene group ;
R¹ to R¹⁸ each independently represent hydrogen atom, a substituted or unsubstituted aromatic hydrocarbon group having 6 to 50 nuclear carbon atoms, a substituted or unsubstituted aromatic heterocyclic group having 5 to 50 nuclear atoms, a substituted or unsubstituted alkyl group having 1 to 50 carbon atoms, a substituted or unsubstituted cycloalkyl group having 3 to 50 nuclear carbon atoms, a substituted or unsubstituted alkoxyl group having 1 to 50 carbon atoms, a substituted or unsubstituted aralkyl group having 6 to 50 carbon atoms, a substituted or unsubstituted aryloxyl group having 5 to 50 nuclear carbon atoms, a substituted or unsubstituted arylthio group having 5 to 50 nuclear carbon atoms, a substituted or unsubstituted alkoxycarbonyl group having 1 to 50 carbon atoms, a substituted or unsubstituted silyl group, carboxyl group, a halogen atom, cyano group, nitro group or hydroxyl group;
**m** and **n** each represent an integer of 0 to 4 and, when **m** and **n** each represent an integer of 2 or greater, atoms and groups represented by R¹⁷ and R¹⁸, respectively, may be a same with or different from each other and may be bonded to each other to form a cyclic structure; and
**q** represents an integer of 1 to 3, and **p** represents an integer of 0 to 2.

2. A bisanthracene derivative according to Claim 1, wherein, in general formula (1), **q** represents 1, and Ar³ represents phenylene group.

3. A bisanthracene derivative according to Claim 1, wherein, in general formula (1), **q** represents 1, p represents 0 or 1, and Ar³ represents phenylene group.

4. A bisanthracene derivative according to Claim 1, wherein, in general formula (1), **q** represents 1, and Ar³ represents naphthylene group.

5. A bisanthracene derivative according to Claim 1, which is a light emitting material for organic electroluminescence devices.

6. A bisanthracene derivative according to Claim 1, which is a host material for organic electroluminescence devices.

7. An organic electroluminescence device comprising a cathode, an anode and an organic thin film layer which comprises one layer or a plurality of layers comprising at least a light emitting layer and is disposed between the cathode and the anode, wherein the organic thin film layer comprises at least one compound selected from bisanthracene derivatives described in Claim 1 singly or as a component of a mixture.

8. An organic electroluminescence device according to Claim 7, wherein the light emitting layer comprises a bisanthracene derivative described in Claim 1 as a host material.

9. An organic electroluminescence device according to Claim 7, wherein the light emitting layer further comprises an arylamine compound.

10. An organic electroluminescence device according to Claim 7, wherein the light emitting layer further comprises a styrylamine compound.
